(19) Europäisches Patentamt
European Patent Office
Office européen des brevets

(11) **EP 4 410 198 A1**

(12) **EUROPEAN PATENT APPLICATION**

(43) Date of publication:
**07.08.2024  Bulletin 2024/32**

(21) Application number: **23020052.9**

(22) Date of filing: **31.01.2023**

(51) International Patent Classification (IPC):
***A61B 5/145*** (2006.01)

(52) Cooperative Patent Classification (CPC):
**A61B 5/14532**

| | |
|---|---|
| (84) Designated Contracting States:<br>**AL AT BE BG CH CY CZ DE DK EE ES FI FR GB GR HR HU IE IS IT LI LT LU LV MC ME MK MT NL NO PL PT RO RS SE SI SK SM TR**<br>Designated Extension States:<br>**BA**<br>Designated Validation States:<br>**KH MA MD TN**<br><br>(71) Applicant: **Nutrix Poland Sp. z o.o.**<br>**30-348 Krakow (PL)** | (72) Inventors:<br>• **PARMAR, Jemish**<br>  **08025 Barcelona (ES)**<br>• **HAHN, Maria**<br>  **4053 Basel (CH)**<br>• **SULOT, Dominika**<br>  **63-600 Kepno (PL)**<br><br>(74) Representative: **Pietruk, Claus Peter**<br>**Mozartstraße 21**<br>**79539 Lörrach (DE)** |

(54) **READ-OUT DEVICE AND CARRIER FOR ANALYTE DETECTION**

(57)  A read-out device comprises an interface adapted to obtain signals indicative of an analyte level of an analyte in a body fluid from a carrier adapted to receive in use the body fluid and to react in a detectable manner with the analyte and/or one or more concomitant substances in the body fluid; and a signal processing stage adapted to process the signals in a manner allowing determination of the level of the analyte in the body fluid; wherein the interface is adapted to obtain at least part of the signal as a time series of signal strengths from the carrier after receipt in use of one type of body fluid selectable from at least two different types with each type containing the analyte in a determinable amount, and the signal processing stage is adapted to assess the analyte level from the time series of signal strengths for either of the types of body fluid received on the carrier.

EP 4 410 198 A1

**Description**

The present invention generally relates to the determination of analytes in body fluids.

In a number of cases, it is desirable to monitor physiological conditions of a living being in certain instances. Monitoring the concentration, presence or absence of certain substances may be advantageous and may in particular help to administer pharmaceuticals, call an emergency, inform the user of a medical condition and so forth.

Examples of analytes that people wish to monitor are analytes indicative of certain diseases, for example proteins and glycosylated proteins, in particular proline-rich peptides, amylase, host-defence peptides, mucins, antibodies such as IgG, IgA, sigA, IgM, secretory IgA, a growth factor such as EGF, NGF, VEGF, IGF or the analyte can be selected from cytokines and chemokines, e.g. IL-1 beta,IL-8, IL-6, MCP-1, CX3CL1, GR0-1 alpha, troponin I, TNF alpha; also nucleic acids such as human DNA, microbial DNA, mRNA, siRNA, micro RNA (miR-125a and miR-200a), and carbonic anhydrase, proteinases, endothelin, lactoferrin, metalloproteinases, chitinase, hexosaminidase, ketones, cholesterol, triglycerides, human choriogonadotropin (HCG), hemoglobin A1C, fructo samine, carbohydrates, tumor markers, nitric oxide radicals, fibronectin, cystatins, neopterin, cytokines, glycated proteins and fats, soluble factor CD44, telomeras, cortisol and dehydroepiandrosterone, estriol, phenylalanine, valine, and lactic acid, zinc-a-2-glycoprotein, haptoglobin, and human calprotectin. The analytes can be indicators or related to different forms of cancer such as oral cancer, pancreatic cancer, gastric cancer, infectual bacterial, viral, fungal and autoimmune diseases, endocrinology, psychiatry, nephrology, caries disease, periodontal disease, gastroesophageal reflux disease (GERD), osteoporosis, kidney disease, cardiology and metabolic diseases such as metabolic acidosis, metabolic alkalosis, and diabetes. Analytes may comprise avian influenza virus, hepatitis B marker HBsAg, cancer marker AFP, human thyroid stimulating hormone, interleukin 8 (IL-8), tumor necrosis factor (TNF -a), cancer biomarker CYFRA21- 1, prostate cancer biomarker PSA, carcinoembryonic antigen (CEA), cardiac troponin I (cTni), C-reactive protein (CRP), prostate cancer biomarker osteopontin (OPN), interleukin-6 (IL-6), cortisol, lyme disease antigen, Alzheimer biomarker amyloid-p, chondroitin sulfate proteoglycan 4, pancreatic cancer biomarker, carbohydrate antigen 19-9 (CA 19-9), prostate specific antigen/1-antichymotrypsin, (PSA-ACT) complex, breast cancer biomarkers human epidermal growth factor receptor 2, human immunodeficiency virus (HIV), bladder cancer biomarker, urinary APOA2 protein, prostate cancer biomarker PSA-ACT complex, D-Dimer, biomarker of venous thromboembolism, breast cancer biomarker EGFR, hemoglobin-Ale, insulin, hormones such as cortisol, androgens, testosterone, estriol, estrogen, progesterone, aldosterone, DHEAS. It is also suggested that analytes relating to fertility periods, pregnancy, labor onset, alcohol concentration, blood glucose concentration are measured as well as indicators that may signal a need for comprehensive HIV testing. In certain cases, a determination shall also be made of analytes relating to anti-epilepsy drugs, bilirubin, liver function markers, toxins or their metabolites, controlled substances or a drug related to abuse, ethanol, therapeutic drugs, anticonvulsants, antipyretic/analgesics, anti-neoplastic agents, anti-bacterial agents, bronchodilators, and cotinine.

Among the many analytes, one particular important analyte is glucose: as an example, where a patient has diabetes, in particular type I diabetes, it is necessary to ensure that the glucose level remains within safe boundaries. As the glucose levels vary strongly during the day, for example because of physical activity of the person, the ingestion of meals and so forth, it is desirable to regularly monitor of glucose levels. Also, glucose levels may be relevant to persons other than diabetes patients, for example to people who want to lose or gain weight, avoid heart disease, metabolic syndrome, hypertension or Type 2 diabetes or athletes who want to optimize their performance. Accordingly, measuring glucose may be of general interest to persons interested in optimizing their physical well-being.

Therefore, a number of methods determining glucose in blood and/or other body fluids as well as for diabetes markers and other analytes have been developed.

EP1 986 007A1 relates to a sensor assembly for body fluids. In the introductory portion, it is noted that it would be preferable to use as little of a patient's blood as possible. While in the introductory portion, blood is explicitly mentioned as a body fluid, explicit reference is also had to saliva. The sensor arrangement comprises a fluid channel with electronic wiring substrates arranged at both sides of the channel. The fluid path is stated to have preferably no or only a limited number of bends or turns. It is stated that the sensor assembly may comprise a liquid chamber to hold a reference electrolyte.

From EP 0 199 484 A2, a handheld pocketable measurement system for extraction and analysis of a liquid in the body is known wherein a measurement is effected in view of the optical characteristic of a liquid such as blood in response to a color change induced by a reagent. It is suggested to calibrate the optical measurement device using color stripes having different shades of gray.

From EP 0 840 122 A, a method and an apparatus for calibrating a sensor element are known. A meter shall include a sensor for receiving a user sample to be measured and a processor for performing a predefined test sequence for measuring a predefined parameter value. A memory is coupled to the processor for storing predefined parameter data values. A calibration code is associated with the sensor and read by the processor before the user sample to be measured is received. The calibration code is used in measuring the predefined parameter data value to compensate for different sensor characteristics.

EP 1 972 270 A1 relates to a method for generating nutritional feedback wherein first, the glycemic response of the individual is determined after consumption of e.g. 50 g of pure glucose, so that thereafter, the response to food consumed can be estimated.

A salivary protein glycosylation test for diagnosis and monitoring of diabetes is known from EP 2 924 436 A1. Kits and lateral flow devices for detecting glycoproteins in a saliva sample are described. It is also suggested that glucose data may be saved, recalled and annotated either in real time or retrorespectively so that a library of reference events can be build and a current glycemic response be compared with a response on earlier occasions.

In KR10-1436915, a device is shown wherein a drop of saliva is placed on a sample part above a conjugation pad and flow is occurring in a direction perpendicular to that, producing well-known color lines. As nitro cellulose membranes are referred to, transportation of saliva seems to take place by capillary action.

US 2016/0313307 A1 relates to a diagnostic test strip for oral samples with a diagnostic test strip that is brought into contact with the tongue of a patient. In the introductory portion, it is stated that the collection of saliva for diagnostic purposes is complicated due to inter alia the relatively high viscosity of salivary fluid and the diverse anatomic dispersion of the salivary glands.

From WO 2014/055559 A1, a microfluidic sensor is known wherein a nano sensor comprises a normal structure with an element comprising 2 metallic structures separated by a gap and a capture agent deposited on a surface of the normal structure specifically binding an analyte.

In the paper "Soft contact lens biosensor for in situ monitoring of tear glucose as non-invasive blood sugar assessment" by Ming Xing Chu, Kumiko Miyajima, Daishi Takahashi, Takahiro Arakawab, Kenji Sano, Shin-ichi Sawada, Hiroyuki Kudob, Yasuhiko Iwasakid, Kazunari Akiyoshi, Manabu Mochizuki, and Kohji Mitsubayashi in Talanta 83 (2011) 960-965, it has been suggested to assess blood sugar in tears. The biosensor was a flexible hydrogen peroxide electrode (Pt working and Ag/AgCl reference/counter electrodes), which was formed on a thin PDMSmembrane (size of 3mm×45mm, thickness: 70$\mu$m). For the purpose of application on eye site, the biosensor utilized a soft PDMS contact lens (base curve radius: 8.6) as a support of the flexible electrode. While it could be shown that the biosensor has a rapid in vitro response to those glucose levels found in tears, to the knowledge of the applicant, the device has not turned into a commercially viable system.

In the paper "Mouthguard biosensor with telemetry system for monitoring of saliva glucose: A novel cavitas sensor" by Takahiro Arakawa, Yusuke Kuroki, Hiroki Nitta, Prem Chouhan, Koji Toma, Shin-ichi Sawada, Shuhei Takeuchi, Toshiaki Sekita, Kazunari Akiyoshi, Shunsuke Minakuchi and Kohji Mitsubayashi in http://dx.doi.org/10.1016/j.bios.2015.12.014, it has been suggested to measure glucose levels in saliva. It is noted that the contents of this document are enclosed fully in this disclosure, in particular with respect to sensor materials and sensing methods for glucose. The salivary biosensor suggested was incorporating Pt and Ag/AgCl electrodes on a mouthguard support with an enzyme membrane is developed and tested. The Pt working electrode is coated with glucose oxydase membrane and integrated in a wireless measurement system. The relationship between the sensor signal and glucose concentration is stated to be "robust"; in more detail, the authors state that saliva glucose concentrations range approximately from 20 to 200 mmol/L in normal and diabetic individuals and closely follow circadian blood glucose fluctuations. Furthermore, they consider that saliva and blood glucose levels correlate reasonably in a sample of individuals, but that a much stronger correlation is observed within the same individual, enabling blood glucose concentrations to be estimated from saliva glucose measurements. Reference is explicitly had to the corresponding quotation of literature found in the article. The authors conclude that a wearable mouthguard glucose sensor produced using micro electromechanical systems (MEMS) techniques would offer promise as a minimally-invasive, painless, continuous, custom-fitted and wireless solution for self-monitoring of glucose. The authors reported that the glucose oxidase is immobilised by entrapment with (PMEH) as otherwise the signal obtained during repetitive use with strongly decrees, given that glucose oxidase is progressively washed off. The authors report that a larger output current is observed with greater electrode surface area; the mouthguard shown covers the first premolar tooth through the third molar tooth on mandible. The authors state that fluctuations in output current were observed whenever the apparatus was disrupted by motion but suggest nonetheless that the mouthguard biosensor has potential utility as tool for real-time noninvasive saliva glucose monitoring within the oral cavity.

It should be noted that a variety of sensors other than glucose sensors have been suggested as well. In the paper "A gold nanoparticle-assisted sensitive SAW (surface acoustic wave) immunosensor with a regeneratable surface for monitoring of dust mite allergens" by Koji Toma, Daisuke Miki, Naoyuki Yoshimura, Takahiro Arakawa, Hiromi Yatsuda, and Kohji Mitsubayashi,. http://dx.doi.org/10.1016/j.snb.2017.04.073, signal amplification in the surface acoustical wave (SAW) is demonstrated for monitoring of airborne house dust mite allergens.

Another biomarker for diabetes mellitus is suggested in the paper "Biochemical Gas Sensors (Biosniffers) Using Forward and Reverse Reactions of Secondary Alcohol Dehydrogenase for Breath Isopropanol and Acetone as Potential Volatile Biomarkers of Diabetes Mellitus" by Po-Jen Chien, Takuma Suzuki, Masato Tsujii, Ming Ye, Isao Minami, Kanako Toda, Hiromi Otsuka, Koji Toma, Takahiro Arakawa, Kouji Araki Yasuhiko Iwasaki Kayoko Shinada, Yoshihiro Ogawa and Kohji Mitsubayashi in Anal. Chem. 2017, 89, 12261-12268.

"A non-enzymatic glucose sensor enabled by bioelectronic pH control" is suggested by Xenofon Strakosas, John Selberg, Pattawong Pansodtee, Nebyu Yonas, Pattawut Manapongpun, Mircea Teodorescu & Marco Rolandi in Scientific Reports | (2019) 9:10844, https://doi.org/10.1038/s41598-019-46302-9. It is noted that the contents of this document are enclosed fully in this disclosure, in particular with respect to sensor materials and sensing methods for glucose. The authors state that known continuous glucose sensors use enzymes with a one to two week lifespan, forcing periodic replacement. The authors note that metal oxides sensors are an alternative to enzymatic sensors with a longer lifetime, but that they do not operate in sweat and tears because they function at high pH > 10. It is suggested to electronically induce a reversible and localized pH change; glucose monitoring at physiologically relevant levels in neutral fluids mimicking sweat is demonstrated. The authors suggest a glucose sensor that some comprises cobalt oxide, palladium (Pd), and silver/silver chloride (Ag/AgCl) contacts electrochemically grown on gold (Au) strips defined on a glass substrate. The $Co_3O_4$ contact is used to sense glucose, the Pd contact is used to change the local pH of the fluid, and the Ag/AgCl contacts act as reference electrodes that balance the half reactions for glucose and pH modulation respectively. Allegedly, the metal oxides sensors do not suffer from limited lifetime due to enzyme degradation over time.

In "Non-invasive platform to estimate fasting blood glucose levels from salivary electrochemical parameters" by Sarul Malik, Harsh Parikh, Neil Shah, Sneh Anand and Shalini Gupta in Healthcare Technology Letters, 2019, Vol. 6, Iss. 4, pp. 87-91, doi: 10.1049/ht1.2018.5081 it is stated that a combination of salivary electrochemical parameters such as pH, oxidation-reduction potential (ORP), conductivity and individual cationic concentrations can be collectively used to estimate fasting blood glucose levels. It is noted that the contents of this document are enclosed fully in this disclosure, in particular with respect to sensor materials and sensing methods for glucose.

In the review "Saliva diagnostics - Current views and directions" by Karolina Elzbieta Kaczor-Urbanowicz, Carmen Martin Carreras-Presas, Katri Aro, Michael Tu, Franklin Garcia-Godoy and David TW Wong published in Experimental Biology and Medicine 2017; 242: 459-472, it is noted that saliva plays an important role in many biological functions such as perception of oral sensations, for example taste, temperature in touch, lubrication, chewing and swallowing and digestion. The authors allege that saliva has the potential to be used for the detection of essential biomarkers and that the salivary diagnostics can be applied in inter alia the fields of medicine, dentistry, pharmacotherapy, epidemiology and bioterrorism. Examples given relate to oral cancer, pancreatic cancer, gastric cancer, infectual bacterial, viral, fungal and autoimmune diseases, endocrinology, psychiatry, nephrology, cardiology and metabolic diseases such as diabetes. The authors refer to different technologies for the salivary diagnostics, e.g. for oral cancer detection, or a test to detect oral human papilloma virus infection that could potentially lead to oral cancer. Specific reference is made to salivary diagnostics and development of microfluidic or microelectromechanical systems. It is stated that technologies enable to measure proteins, DNA, transcripts (mRNA) and electrolytes and small molecules in saliva. They state that tools including electrochemical sensing, on-chip security-PCR, high throughput DNA microArray, surface plasmon resonance based arrays fiber optic sensors and microchip-based electrophoretic immunoassays are being developed.

In "Effect of Diabetes Mellitus Type 2 on Salivary Glucose - A Systematic Review and Meta-Analysis of Observational Studies" by Paulo Mascarenhas, Bruno Fatela and Isabel Barahona, PLOS ONE 1 July 2014, Volume 9, Issue 7, e101706, it is stated that a meta analysis shows the significant overall relationship between salivary glucose concentrat ion and associated glycemia/be HB A1c values.

In the paper "Salivary Glucose as a Diagnostic Marke for Diabetes Mellitus" by Komal Smriti, Keerthilatha M. Pai, Vineetha Ravindranath, BDS, MDS1, Srikanth Gadicherla, and Kalyana Chakravarthy Pentapati, it is reported that salivary glucose concentrations above a certain level indicate that the person has uncontrolled diabetes mellitus.

In the paper "Wearable salivary uric acid mouthguard biosensor with integrated wireless electronics" by Jayoung Kim, Somayeh Imani, William R. de Araujo, Julian Warchall, Gabriela Valdés-Ramírez, Thiago R.L.C. Paixão, Patrick P. Mercier and Joseph Wang, Biosensors and Bioelectronics 74 (2015) 1061-1068, it is stated that the realization of wearable biosensors for real-time monitoring of chemical markers is limited by the small number of demonstrated target analytes and the lack of integrated wireless data transmission; challenges such as power consumption and size of wireless sensor systems are explicitly mentioned. Explicit reference is had to a radiofrequency identification (RFID) wireless sensor tag with potentiometric input, stating that the tag is too large for integration in anatomically sized platforms, powered by a 3V battery and requiring a larger reader device to be positioned in close proximity to the tag for data readout. Thus, reference is made to a wireless parametric circuit paired with a Bluetooth low energy communication system on chip for miniaturized and low power operation, fully integrated into a salivary metabolite mouthguard biosensor for continuous real-time amperometric monitoring. The authors refer to an enzyme electrode for the detection of uric acid. It is stated that on the enzyme a layer of polymerized OPD was deposited by electric polymerization to minimize bio following an interference effects from saliva constituents.

In the paper "Noninvasive glucose monitoring using saliva nano-biosensor" by Wenjun Zhang, Yunqing Du and Ming L. Wang inSensing and Bio-Sensing Research 4 (2015) 23-29, the authors report that salivary glucose and blood glucose levels share the same fluctuation trend, but that the correlation between them is individual dependent and a time lag exists between the values from blood and saliva, although the correlation between 2 glucose values at fasting is constant for each person. It is noted that the contents of this document are enclosed fully in this disclosure, in particular with respect to sensor materials and sensing methods for glucose. The authors reported good correlation of glucose levels in saliva and blood before and 2 hours after glucose intake. The authors refer to an on-chip disposable nano biosensor providing a painless test methodology which is disposable in order to eliminate extensive cleaning or electron pretreatment between measurements.

In the paper "On-chip highly sensitive saliva glucose sensing using multilayer films composed of single-walled carbon nanotubes, gold nanoparticles, and glucose oxidase" by Wenjun Zhang, Yunqing Du and Ming L. Wang, Sensing and Bio-Sensing Research 4 (2015) 96-102, the authors suggest a disposable low-cost biosensor on a single chip using a working electrode functionalized through a layer-by-layer assembly of singlewall carbon nanotubes and multilayer films composed of chitosan, gold nanoparticles and glucose oxidase to obtain high sensitivity and accuracy. It is stated that an effective immobilization of enzymes onto an electrode surface is one of the main factors that affect the sensing performance of an enzyme biosensor. It is also stated that chitosan is widely applied to immobilized biomolecules, especially in the assembly of enzymes and fabrication of amperometric biosensors. It is stated that the glucose sensors described detect glucose levels by keeping track of electrodes directly transferred through glucose oxidase to the electrode surface. It is noted that the contents of this document are enclosed fully in this disclosure, in particular with respect to sensor materials and sensing methods for glucose.

According to the paper "Correlation of salivary glucose level with blood glucose level in diabetes mellitus" by Shreya Gupta, Meghanand T Nayak, JD Sunitha, Geetanshu Dawar, Nidhi Sinha and Neelakshi Singh Rallan in JOMFP, 2017, Vol 21, Issue 3, p. 334-339, salivary glucose levels were found to be higher in uncontrolled and controlled diabetes groups than in nondiabetic groups. It is reported that a statistically highly significant correlation was found between fasting saliva glucose and fasting blood glucose in all groups examined. It is concluded that salivary glucose levels can be used for monitoring the glucose level in diabetes mellitus.

In "Pacifier Biosensor: Toward Noninvasive Saliva Biomarker Monitoring" by Laura Garcia-Carmona, Aida Martin, Juliane R. Sempionatto, Jose R. Moreto, Maria Cristina González, Joseph Wang, and Alberto Escarpa in Anal. Chem. 2019, 91, 13883-13891, a chemical wearable sensor for newborn monitoring is suggested which is fully integrated into a pacifier operating as a portable wireless device. Specific reference is made to monitoring chemical biomarkers for rapid control of metabolic diseases such as diabetes. The authors cite attempts to measure glucose optically through the skin, for example finger and earlobe but note that these technologies still face several limitations such as nonspecific absorption and thermal noise for external optical sensors along with low sensitivity for colorimetric ones. The authors are aware that devices inside the mouth might be uncomfortable, may cause problems because of the direct contact of sensor materials such as the potentially harmful protective (anti-fouling) layers and so forth. The authors describe studies and sample collection for evaluation of the device wherein prior to saliva sampling, teeth have to be brush using toothpaste and carefully rinsing the mouth with plenty of water avoiding toothpaste residue. The authors state that a new modified electrode was used for each experiment. The authors report that the limit of detection (LOD) and quantification (LOQ) were good enough to quantify the regular levels of glucose in diabetes patients.

The paper "Sensing of Salivary Glucose Using Nano-Structured Biosensors " by Yunqing Du, Wenjun Zhang and Ming L. Wang, suggest detecting salivary glucose using a layer by layer self-assembly of singlewall carbon nanotubes, chitosan, gold particles and glucose oxidase onto a screen-printed platinum electrode is suggested. The authors also give an overview of screen-printed glucose biosensors; for measuring in saliva, only nanoparticles and glucose oxidase sensors are referred to. One object of the human subject studies the authors refer to is stated to be screening for diabetes by measuring saliva glucose levels at fasting.

From CA 2 732 786 A1, a method and apparatus for non-invasive analysis of saliva is known it is suggested that a reagent on the apparatus is exposed to a saliva sample and interacts with certain constituents thereof to produce a color change made visible on the apparatus. It is suggested that for glucose testing, the reagent chemistry can comprise the enzymes glucose oxidase and peroxidase, a chromogenic that produces a die when reacted with hydrogen peroxide, a buffer to provide an appropriate pH for both enzymes, one or more surfactants that enhance color development and absorption of the fluid being tested and other inert ingredients for enzyme stabilization or termination of interfering substances. It is noted that other testing is suggested such as testing for alcohol levels, ketone level testing, cholesterol level testing, cortisol level testing, illicit drug level testing, pregnancy testing, salicylate level testing. It is suggested that the apparatus for conducting the noninvasive analysis of saliva may comprise a lollipop like structure including a stem and a head, the stem being selectively movable; in one of the position achieved by moving, the user can bring the stem in contact with his mouth. It is noted that the contents of this document are enclosed fully in this disclosure, in particular with respect to sensor materials and sensing methods for glucose.

In the paper "The Secretion, Components and Properties of Saliva" by Guy H. Carpenter in Annu. Rev. Food Sci. Technol. 2013. 4:267-76, the neural regulation of the delivery of secretion in terms of fluid, proteins and ion secretion is reviewed.

In the paper "Salivary Markers for Periodontal and General Diseases" by Stepan Podzimek, Lucie Vondrackova, Jana Duskova, Tatjana Janatova, and Zdenek Broukal. in Disease Markers, Volume 2016, Article ID 9179632, http://dx.doi.org/10.1155/2016/9179632, it is noted that saliva is a source of markers for a number of periodontal diseases; the authors refer in particular to the following substances that may be indicative for certain diseases: proteinases, endothelin, lactoferrin, metalloproteinases, chitinase, hexosaminidase, nitric oxide radicals, fibronectin, cystatins, neopterin, cytokines, glycated proteins and fats, soluble factor CD44, telomeras, cortisol and dehydroepiandrosterone, estriol, phenylalanine, valine, and lactic acid, zinc-a-2-glycoprotein, haptoglobin, and human calprotectin). The authors also indicate to what diseases or specific conditions of the patient these substances relate. Specifically, with respect to diabetes mellitus, the authors indicate that the glycated hemoglobin level in blood has long been used as a marker of metabolically poorly controlled diabetes and that even substance levels in saliva correlate with the glycated hemoglobin level in plasma.

In the review paper "Recent advances in electrochemical non-enzymatic glucose sensors -a review " by Dae-Woong Hwang, Saram Lee, Minjee Seo, Taek Dong Chung in Analytica Chimica Acta 1033 (2018) 1-34, mechanisms of electrochemical glucose detection based on a variety of materials ranging from platinum, gold, metal alloys/other term, non-previous transition metal/metal oxides to glucose-specific organic materials are reviewed. It is noted that the contents of this document are enclosed fully in this disclosure, in particular with respect to sensor materials and sensing methods for glucose. The authors note that blood glucose monitoring based on glucose oxidase and glucose dehydrogenase is impaired by e.g. maltose, requiring caution in any potential drug interaction before use and that glucose dehydrogenase systems show bias in the measured glucose level due to reactivity towards sugars other than glucose. The authors also advise that the sensitivity of enzymatic glucose sensors largely depends on the activity of the immobilized substances; thus, non-enzymatic sensors without biological functional units are suggested to be advantageous in terms of structural simplicity and quality control for mass production. It should be noted that the mechanisms of direct glucose oxidation with metallic redox centers in non-enzymatic glucose electrochemical detection are discussed; the authors then relate to nanostructures for electric catalytic glucose detection, stating that they see three important aspects of nanostructures electrode materials, namely generation of electric catalytic active sites, enlargement of surface area informational nano space enclosed by conducting surfaces. The authors note that nano pores may give rise to increased cross-sensitivities. With respect to non-enzymatic glucose sensors, the authors relate to the use of the following catalyst matters: gold, palladium-nano porous gold, platinum-nano porous gold, platinum-gold, platinum-ruthenium, platinum-iridium, platinum-palladium, platinum-cadmium, nickel-copper, palladium-copper-platinum, platinum-copper, platinum-nickel, platinum-led, platinum-delirium, cobalt at platinum, platinum-bismuth, nickel, nickel-gold, nickel oxide, nickel oxide-platinum, nickel oxide-carbon, nickel oxide-zinc oxide, nickel oxide-copper, $Ni(OH)_2$, nickel-metal-organic coordination polymer, copper,$Cu_2O$, $CuO$, $Cu@Cu_2O$, $Co_3O_4$, $CoO_x$, $Fe_2O_3$, $Fe_3O_4$, $Fe@ZnO$, $MnO_2$, $MnO_2/Cu=$, $Mn_3O_4$, $SnO_2$, Mn-Cu, SnO2, Mn-Cu, $NiCo_2O_4$, $Fe_2Ni_2N$,$Fe_3N$-$Co_2N$,Co3N, CoP, $Ni_2P$, NiCoP, $Ni_2FeS_4$, CoSe, CoS,$Ni_7S_6$, tin oxide, and in different morphologies such as porous, nano porous or microporous films, submonolayers, thin films, nano wire, micro tubes, bismuth /platinum nanoporous micro -pillar arrays, lamellar ridge-like morphologies, nanocorals, dendrites, arrays, thin coating, coating, microparticles, hollow nanocrystals, nano thorn array, micro flowers, nano flowers, and on substrates/electrodes such as compact disc-recordable, carbon powder/plastic carbon electrodes, dendrite like gold, bar and doped diamond/CCM wafer, titanium plate, mesoporous carbon/glassy carbon electrode, Vulcan carbon/glassy carbon electrode, Au-Si-Wafer, SPCE, Au disk, Au wire, glassy carbon electrodes, ITO glass, fluoride doped tin oxide glass, diatom biosilica-graphene oxide nanorod/carbon sheet, multi walled carbon nanotubes-cryogel/Au disk, poly(aniline)/glassy carbon electrode, graphene, reduced graphene oxide, graphene foam, carbon powder, carbon paste, ionic liquid, screen-printed carbon electrode, copper nano wire/glassy carbon electrode, sandpaper, zeolitic imidazolate framework- 8/screen-printed carbon electrode, N-doped carbon/glassy carbon electrode, multiwall carbon nanotubes/Ta foil, copper foam, carbon felt, platinum disc, graphite wafer, ZnO NR/ Si wafer,Carbon cloth, Ti mesh, Cu foam, bi-C, which will walled carbon nanotubes/Ta foil and chitosan. Furthermore, selective binding-based electrochemical non-enzymatic glucose sensors are mentioned such as boronic acid, pyridinic N, Amide, -COOH, Amide, -$NR_2$, - OH, -Phenyl, -alkyl. It is stated that noble metal and alloy-based sensors are advantageous in that they can operate in neutral pH conditions reasonably well, but that they show inferior sensitivity, anti-infective interference and stability compared to transition metal counterparts. The authors suggest that the use of iron-selective protective membranes, nano engineering and alloying could alleviate this problem.

In the paper "Glucose estimation and the salivary secretion of diabetes mellitus patients" by Panda Abikshyeet, Venkatapathy Ramesh and Nirima Oza in Diabetes, Metabolic Syndrome and Obesity: Targets and Therapy, 2012:5 149-154, a study is presented showing a statistically significant correlation coefficient between 0 and glucose in the salivary glucose in both healthy control subjects and patients newly diagnosed with type II diabetes mellitus.

An assessment of different saliva collection methods for detecting and quantifying biomarker levels in saliva can be found in the paper "Effect of Saliva Collection Methods on the Detection of Periodontium-Related Genetic and Epigenetic Biomarkers-A Pilot Study" by Ping-ping Han and Saso Ivanovski in Int. J. Mol. Sci. 2019, 20, 4729; doi:10.3390/ijms20194729. The authors note that the composition of saliva may vary considerably according to a flow rate, the type of stimulation and time of day, while salivary flow rate varies among individuals.

In the paper "influence of saliva on the oral microbiota" by Philip D. Marsh, Thuy Do, David Beighton and Deirdre A. Devine in Periodontology 2000, page 80-92, it is stated that over 1,000 proteins have been detected (the salivary proteome), many of which are glycosylated, although perhaps only 10% of these are present in high abundance. Among the most prevalent proteins are the proline-rich peptides, amylase, host-defence peptides, mucins, secretory IgA and carbonic anhydrase.

It has also been attempted to quasi-continuously monitor salivary glucose levels in a non-obtrusive manner, e.g. in the paper "Graphene-based wireless bacteria detection on tooth enamel" by Manu S. Mannoor1, Hu Tao2, Jefferson D. Clayton, Amartya Sengupta, David L. Kaplan, Rajesh R. Naik, Naveen Verma, Fiorenzo G. Omenetto and Michael C. McAlpine in nature communications 3 7 6 3, doi: 10.1038/ncomms1767. The authors suggest to print graphene onto water-soluble silk and to include a resonant coil eliminating the need for onboard power supply and external connections. It is noted that the authors suggest a transferral the graphene/electrode/silk hybrid structure to biomaterial followed by functionalization.

In the paper "Highly sensitive non-enzymatic electrochemical glucose biosensor using a photolithography fabricated micro/nano hybrid structured electrode" by Che-Wei Hsu, Fang-Ci Su, Po-Yu Peng, Hong-Tsu Young, Stephen Liao, Gou-Jen Wang, in Sensors and Actuators B 230 (2016) 559-565, a non-enzymatic glucose biosensor based on a simple photographic process has been suggested. The authors suggest to use a gold nanoparticles-based sensor with an area of 1x1 cm$^2$ placed in the mouth.

In the paper "Fasting salivary glucose levels is not a better measure for identifying diabetes mellitus than capillary blood glucose levels: comparison in the Ghanian population" by Richard K. D. Ephraim, Enoch Odame Anto, Emmanuel Acheampong, Linda Ahenkorah Fondjo, Richmond B. Barnie, Samuel Asamoah Sakyi and Ambrose Asare, Heliyon 5 (2019) e01286, doi: 10.1016/j.heliyon.2019, e01286 it has been stated that fasting salivary glucose levels increase with increasing blood glucose levels; the authors note that the diagnostic accuracies and specifities for fasting salivary glucose, fasting serum glucose and fasting capillary whole blood glucose levels are comparable suggesting that salivary blood glucose could be used for diagnosis and monitoring diabetes, but that when utilizing salivary glucose to monitor diabetes patient, factors such as a water intake, the environment sector, mood and anti-diabetes medication must be considered.

US 2007/0106138 A1 relates to an intraoral apparatus for non-invasive blood and saliva monitoring and sensing and suggests a controlled sample sampling oral device implanted or inserted into an oral cavity, built onto a prosthetic tooth crown, dental plate, braces, a dental implant or the like stating that the devices should be replaced as needed, but suggesting that the oral devices might require replacement at long intervals of weeks or months. It is noted that in the application, a plurality of block diagrams are shown indicating the use of modules such as a microprocessor, Asics, discrete ICs, communication based on infrared, Bluetooth, WIFI or a combination thereof and that use of the device is considered inter alia for chrono-therapy sample sampling e.g. for cancer, arthritis as well as for diabetes.

US 2008/0020477 A1 relates to salivary glucose monitoring and more particular suggests various devices and methods of processing a saliva sample obtained from a mammal, particularly a human.

US 2016/00/97734 A1 describes a salivary glucose monitoring system with a glucose sensor obtained by functionalizing singlewall carbon nanotubes deposited onto the surface of a working electrode in a 3 electrode electrochemical detector by depositing layers of polmeres, metal nanoparticles and glucose oxidase & onto the nanotubes. The sensor is stated to be used as a disposable, single use device or as part of an analytical system such as a microfluidic system. A layout for an antioxidant detector including an electrochemical sensor array, a potentiostat, a signal processing area and a wireless transmitter to a smart phone are shown. The suggested device has an inlet and a microfluidic sample preparation.

US 2019/0025131 A1 refers to salivary glucose measurement devices and methods. The detection mechanism describes comprises pipette transfer onto a sensor of a saliva sample collected by natural salivating.

WO 2017/058806 A1 describes wearable sensor arrays. The wearable sensing platform suggested may include a flexible printed circuit board to enable the variable sensing platform to conform to a portion of the users body. In an example given, wearable sensor array is a smart wristband. Specific reference to the analysis of sweat is given.

Regarding the construction of elements such as carrier strips, consumables, electrodes and so forth, it has been suggested in WO 2013/131130 A1 to use organic thin film transistors for sensing glucose levels.

The organic thin film transistor has a source electrode, a drain electrode connected to the source electrode via an organic semiconductor layer, and a gate electrode separated from the semiconductor layer by a dielectric layer. It is suggested that the gate electrode consists of a porous matrix, for example a sulfonated tetrafluoroethyleric-based fluoropolymercopolymer, such as ethanesulfonic acid, 2-[ 1-[ difluoro[ (1, 2,2-trifluoroethenyl)oxy ]methyl]-1, 2,2,2- tetrafluoroethoxy]-1, 1,2,2-tetrafluoro-, polymer with 1,1, 2,2-tetrafluoroethene. It is also suggested to use poly(4- vinylphenol) for the dielectric layer, for example lithium perchlorate doped poly(4-vinylpyridine). Other materials suggested for the dielectric layer comprise polyimide and poly(methyl methacrylate (PMMA). Regarding the semiconductor layer, it is suggested to use polyacetylenes, porphyrins, phthalocyanins, fullerenes, polyparaphenylenes, polyphenylenevinylenes, polyfluorenes, polythiophenes, polypyrroles, polypyridines, polycarbazoles, polypyridinevinylenes, polyarylvinylenes, poly (pphenylmethylvinylenes), including derivatives and co-polymers thereof. Specific reference is had to poly(9,9-dioctylfluorene-2,7-diyl-co-bis-N,N-(4- butylphenyl)-bis-N,N-phenyl-1, 4-phenylenediamine ), poly(9, 9-dioctylfluorene-2, 7 -diyl-cobenzothiadiazole), poly(3-hexylthiophene), (6,6)-phenyl-C61 -butyric acid methyl ester and poly(2-methoxy-5-(2'-ethyl-hexyloxy)-1, 4-phenylene vinylene ). It is suggested that an enzyme such as glucose oxidase is located either in the gate electrode or in the dielectric layer. Other materials that are explicitly referred to comprise poly(3-hexyl-thiophene) and indium tin oxide ITO. Methods to produce such a device are described and it is suggested to use the device to sample a bodily fluid, in particular saliva by bringing the gate electrode in contact with the analyte. Note that a substrate may be glass, low-cost plastics or paper. A diagram is shown indicating the drain current for various glucose concentrations versus times up to 1200 seconds.

WO2019/14415581 relates to a long-term continuous wireless intraoral monitor stating that caries disease is directly related to low salivary pH, but that no no objective tool of measuring a patient's risk level exist as a one time reading of salivary pH would not give sufficient information regarding the conditions of the oral cavity or the development of carious disease. Also, it is stated that caries leads to poor oral health and that this has been shown to be related to cardiovascular disease and diabetes, although other disorders are mentioned as well. For measuring, it is suggested to install the sensor on a molar by a dentist.

US 9766199 B2 relates to an organic thin film transistor based sensor device, the device including a gate electrode; a dielectric layer; a semiconducting layer including at least one organic compound; a source electrode;a drain electrode; a substrate; and an enzyme located in or forming part of any of the aforementioned elements, wherein the device further has a top gate bottom contact configuration such that the gate electrode is disposed above the dielectric layer, and the dielectric layer is disposed above the semiconducting layer, and wherein the gate electrode comprises a porous matrix comprising a sulfonated tetrafluoroethylene-based fluoropolymercopolymer.

WO2018/000012 also relates to organic thin film transistors, their preparation and their use in glucose sensing and in particular, discussing the temporal response of the device and stating that by reducing the thickness of the dielectric layer, the response time thereof can be improved.

In WO 2019/218011 A 1, a further organic thin film transistor is suggested comprising a wicking layer for receiving a liquid sample; within the porous layer, enzymes shall be disposed facilitating the generation of charge carriers in the thin film transistor. The documents states that the wicking layer is advantageous as the wicking effect improves incorporation of a liquid sample into the device ensuring delivery of a standard volume of liquid sample. Furthermore, it is stated that the analyte is exposed to a high degree to the enzyme retained within the wicking layer, resulting in a rapid detection of the analyte.

From EP 3 220 138 A1, it is known to self-calibrate a chemical sensor and it is suggested that self-calibration woll reduce the variation in measured values and will help to measure concentrations accurately.

From JP 2002 122562, a bisosensor for saliva is known composed of at least a determination electrode and a counter electrode, a saliva reaction part composed of an enzyme and an electron acceptor and a detection part which measures a reaction. A sugar concentration in the saliva is determined and it is stated that a sugar concentration which is related to a blood sugar value can be determined painlessly, in a short time and simple.

In US 2007/0233 395A1, a diagnostic meter is suggested including a meter assembly that communicates with a partner device such as a PC. It is suggested that test media -such as carrier strips- from different manufacturers or from different manufacturing lots may respond differently to analyte in a sample. In order to obtain accurate results, the electronic meter is suggested to be calibrated with one or more calibration parameters that correlate the signal response from a particular brand or lot of test media to a standardized reference. It is stated that the user may provide the calibration data to the meter by inserting a code chip into a corresponding port on the meter. It is also suggested that the meter may be conFig. d to read a calibration code on a diagnostic test strip or that alternatively, the meter may only be provided with strips corresponding to a preprogrammed set of calibration data for use with the meter. the method suggested, in particular for calibration parameter transfer into the diagnostic meter, are included herein fully by reference.

In WO 2005/080970, it is suggested to provide more than one level of glucose level calibration to allow more accurate calibration of each result so that more accurate blood glucose testing is allowed and the need for batch calibration reduced.

In WO 2017/116503, a method for real-time calibration of a glucose sensor for measuring the level of glucose in a body of a user is suggested, with the sensor including physical sensor electronics, a microcontroller, and a working electrode, the method comprising measuring, by said physical sensor electronics, the electrode current (Isig) for a working electrode; obtaining a blood glucose (BG) value for said user; calculating, by said microcontroller, an expected calibration factor (CF) value based on said glucose sensor's age; and calculating, by said microcontroller, a calibrated sensor glucose (SG) value associated with said Isig based on said CF and BG values.

While it is obvious from the above that certain analytes can be measured in a variety of ways and in a variety of different body fluids, for specific analytes certain measurements methods have been established to be particularly reliable, for example under laboratory conditions. For example, it is common to test for analytes such as glucose in blood in a clinical set up; values established in this manner can be considered the "gold standard" and any measurement outside such clinical set up should give the same results, even if obtained with body fluids other than blood. However, testing for analytes such as glucose in blood is frequently considered bothersome as either a sensor has to be implanted or at least some connection to the blood circulation piercing the skin is needed. For example, it has been suggested in the prior art to use a sensor for monitoring glucose levels that pierces the skin of the patient to sense glucose levels in blood. These devices commonly are attached to the arm of a patient where movement of skin and physical strain on the device are rather low. However, despite miniaturization, many users dislike that the device will be perceptible by others. Also, problems may occur where the skin is pierced, typically necessitating an exchange of the device every 1 to 2 weeks. For a person just interested to monitor glucose levels without a medical need to do so, this may be completey unacceptable.

Accordingly, where such connection is at least semipermanent, a danger of infections exists; where blood is obtained for each measurement, the measurement is frequently considered painful.

Thus, it is desirable from a practical point of view to allow for measurements by different methods, in particular relying on fluids that can be obtained painlessly. However, a further problem exists in that the methods typically rely on a chemical reaction with the analyte; accordingly, the response to a given concentration of analyte may vary significantly from batch to batch, e.g. due to aging of a batch and/or for different fluids. Therefore, measurements relying on different fluids will be even less reliable.

Furthermore, it would be desirable to have a device that is less obtrusive and still allows a reliable monitoring of physiological conditions. It has already been suggested that the blood glucose levels correlate well with glucose levels in other body fluids. Accordingly, efforts have already been made to measure glucose levels in body fluids other than blood.

It is an object of the present invention to improve measurements of analytes in body fluids. This object is achieved by the independent claims. Preferred embodiments are described in dependent claims.

According to a first idea of the present invention, a read-out device comprising an interface adapted to obtain signals indicative of an analyte level of an analyte in a body fluid from a carrier adapted to receive in use the body fluid and to react in a detectable manner with the analyte and/or one or more concomitant substances in the body fluid; and a signal processing stage adapted to process the signals in a manner allowing determination of the level of the analyte in the body fluid is suggested, wherein the interface is adapted to obtain at least part of the signal as a time series of signal strengths from the carrier after receipt in use of one type of body fluid selectable from at least two different types with each type containing the analyte in a determinable amount and the signal processing stage is adapted to assess the analyte level from the time series of signal strengths for either of the types of body fluid received on the carrier.

Therefore, the invention has realized that despite the variability frequently encountered in analyte measurements, by acquisition of a time series of values (rather than determination of only a single value) and by subsequent assessment of the time series, a given analyte can be determined even when using one and the same (disposable) carrier with different body fluids. It will be understood that while the same analyte will cause the same chemical reaction or some of the same chemical reactions, its concentration - and hence frequently a concentration dependent signal strength such as e.g. a color intensity will be different from fluid to fluid. Furthermore, using different body fluids may cause different cross reactions that may increase or decrease the strength of a signal obtained by the chemical reactions between the analyte and the reagents. Also, using disposable carriers that comprise reagents for reaction with the analyte can be expected to show significant variations in overall signal strength from batch to batch. It can be anticipated that due to such variations, for a given analyte and a given pair of different body fluids, the signal strengths obtained for some concentrations with a first body fluid may be as large as the signal strengths obtained for different concentrations with the other body fluid.

However, it has been found that despite such overlap of the signal strengths themselves, the behavior of the signal strength over time allows to identify both the fluid applied to the carrier and the concentration of an analyte in an unambiguous manner.

Accordingly, the present invention suggests that one and the same type of carriers can be used for different body fluids if the analyte in both types of fluids is determined in a manner where enough - and correct - information is obtained from the measurement and to this effect, a time series of values is obtained and evaluated as such in an assessment stage.

It can be anticipated that this is helpful for a number of analytes which can be examined using the device of the present invention as a number of analytes are present in different body fluids. Such analytes may comprise analytes indicative of certain diseases, for example glucose, cortisol, lactic acid, cytokines, hemoglobin A1C, cholesterol, triglycerides, troponin I, human choriogonadotropin (HCG), carbohydrates, phenylalanine, ketone, thyroid hormones, C-reactive protein (CRP), insulin and therapeutic drugs. It is explicitly noted that - while applicant reserves the right to restrict the application to any, some or all of these analytes- this list of analytes is not intended to restrict the possible analytes per se; rather, the purpose of the list is to indicate that there is a broad range of possible applications and a great number of chemically different analytes without meaning to exclude other analytes.

It will be understood that more than one analyte could be determined using the read-out device er of the invention, for example, carriers to be used with the device could be diabetesrelated, or could be carriers for pregnancy tests and/or stress indication. If the read-out device can be used for more than one analyte, it would be possible to make use of the same type of carrier with different body fluids for only one analyte, but different carriers could be used for different analytes, e.g. a first type of carrier for measuring glucose in either saliva or blood and a second type of carriers for detection of stress-level indicator cortisol. In such an example, chronamperometry could be used in both cases, but the electrodes of the respective types of carriers would be coated with different reagents and the models used for evaluating a cry nonparametric series in order to determine an analyte concentration would also be different. This context, it should also be understood that while reference in the application is typically made to an analyte and detecting an analyte such as glucose, for a number of cases and analytes, it would also be possible to detect concomitant substances rather than the analyte itself. However, for the sake of linguistic simplicity, typically, reference is had only to the analyte itself and not its concomitant substances also usable for detection and analysis.

One particularly important and preferred analyte is glucose. In the context of the invention, glucose can be measured both for a healthy person intending to optimize her well-being and for person a patient having diabetes or a diabetic condition. Such "diabetic condition" may include pre-diabetes and diabetes. Diabetes may in particular be Type 1 diabetes or juvenile-onset diabetes, which is an auto-immune disease characterized by destruction of the pancreatic cells that leads to a total or near total lack of insulin. Furthermore, the diabetes disease might also be a type 2 diabetes sometimes referred to as "non-insulin-dependent diabetes" or "adult-onset diabetes", where the body does not respond to insulin, though it is present.

The carrier may be a solid support art and include, without limitation, nitrocellulose, natural polymeric carbohydrates and their synthetically modified, cross linked or substituted derivatives, such as agar, agarose, cross linked alginic acid, substituted and cross linked guar gums, cellulose esters, especially with nitric acid and carboxylic acids, mixed cellulose esters, and cellulose ethers; natural polymers containing nitrogen, such as proteins and derivatives, including cross-linked or modified gelatins; natural hydrocarbon polymers, such as latex and rubber; synthetic polymers which may be prepared with suitably porous structures, such as vinyl polymers, including polyethylene, polypropylene, polystyrene, polyvinylchloride, polyvinylacetate and its partially hydrolyzed derivatives, polyacrylamides, polymethacrylates, co-polymers and terpolymers of the above polycondensates, such as polyesters, polyamides, and other polymers, such as polyurethanes or polyepoxides; and mixtures or copolymers of the above classes, such as graft copolymers obtained by initializing polymerization of synthetic polymers on a preexisting natural polymer.

In various embodiments related to glucose detection, the methods are based on the oxidation of glycoproteins in a body fluid such as saliva or urine, for example using sodium metaperiodate, and then detecting the aldehydes generated during oxidation using a chemical detection method. In various of embodiments related to glucose detection, the method is based on the finding that the specific glycoproteins oxidized in the sample, primarily sialic acid and fucose, provide an accurate measure of glycemic control when compared to traditional monitoring tools such as HbAlc and fructosamine.

An advantage of using body fluids other than blood for an assessments of glycemic status is that there are many instances in which using blood is disadvantageous, e.g. because of patient age or attitude toward fingersticks or venipuncture, or because of hygiene issues in certain areas such when a diabetes patient is camping in a remote area. The invention allows to easily switch from one body fluid to another in such situations and also allows to select the body fluid currently most convenient to the user.

The carrier itself will typically be disposable; since the signal generation is based on electrochemical reactions that take place using a body fluid, the carrier will accordingly be a disposable electrochemical biosensor. The carrier also is most likely adapted to microfluidically transport the body fluid from a body fluid application zone to an observation area spatially separated from the body fluid application zone where a signal can be obtained. The read-out device can be constructed such that body fluid can be applied while the carrier already is inside the read-out device, but preferably, the carrier strip can be inserted easily into the read-out device once the body fluid has been applied. This avoids contamination of the read-out device by inattentive patients. The read-out device may have an opening formed to allow easy insertion of a read-out device , e.g. the entrance can be funnel-shaped.

The disposable carrier may be adapted to effect the microfluidic transport of the body fluid by way of capillary forces, e.g. using a wick or a narrow microfluidic channel. This way of transport obviates the need to dilute the sample, thus reducing the effect of varying amounts of body fluid applied to the application zone. Also, structures such as wicks might be helpful to destroy foam or froth in the body fluid collected, particularly in saliva.

Using capillary forces- with a wick or by providing sufficiently small fluid channels - has been found to be possible despite the difference in viscosity between different body fluids such as urine (low viscosity), blood (higher viscosity, that is a viscosity higher than the low viscosity of urine) and saliva (viscosity varying). It is noted in this context that saliva often has a matrix that makes it difficult to analyze an analyte in saliva as the matrix can have a variable viscosity, sometimes being much more viscous than traditional samples such as blood serum or plasma. It would be possible to form the carrier entrance itself as an opening of a capillary tube or channel, as this allows to "suck in" by capillary forces the right amount of fluid from a drop of blood, urine, saliva or directly from saliva in the mouth.

It would be possible to add reagents during the transportation, for example if the reaction between analyte and reagent to create a detectable signal takes time, e.g. because first, some intermediate stage needs to be produced, although in a preferred embodiment, reagents are added in the target zone only, because then the onset of the reaction can be observed; observing the onset of a reaction gives valuable information to a time series because it at least guarantees that the signal "decay" is recorded completely - hence this is preferred for the present invention. The tip of the carrier could even be formed like a skin-piercing tip so that blood can be directly retrieved from a finger while for other body fluids, the piercing function would not adversely affect use.

For the precise determination of a total analyte concentration, calibration parameters can use data storage and transmission technologies such as QR codes or RFID tags that are on a package of carriers sold to a user. In a further embodiment, the carriers might be manufactured in a manner that allows the technology to be free from the necessity of calibration.

It is preferred if the carrier is adapted to react with an analyte in the at least two types of body fluid and the interface is adapted to obtain the signals optically, electrochemically, in particular by potentiometry, preferably (chrono)amperometrically and/or by electrical impedance spectroscopy.

If the signal is read out optically, the reaction chosen will have an optically detectable signature, for example because a change in color of reagents can be observed or a spectroscopic signature such as an absorption or fluorescence are altered; the carrier will have an observation area where the reaction takes place or to which reaction products are transported, e.g. by capillary transport, flushing or the like. It would be possible to optically establish the presence of a fluid on the carrier per se or at a specific area on the carrier, such as a fluid frond having reached a given zone the microfluidic channel. This allows to measure the specific front propagation speed and thus helps or allows to decide which body fluid has been applied. When intermediate area or the observation area is observed with an optical sensor arrangement, the optical sensor arrangement can comprise a single light sensitive sensor, typically provided with a spectroscopic filter so that light only from certain wavelength ranges is detected with the sensor. In an alternative embodiment, multiple light sensitive sensors can be provided, preferably each having a sensitivity which is different for each wavelength. Furthermore, one or more light sources such as LEDs of one or more colors can be provided to illuminate the observation area; where multiple LEDs are provided, the illumination with the multiple LEDs could be simultaneous, but it generally is preferred to cycle through the multiple LEDs so that at any time, the observation area is illuminated with light from one LED only, which makes signal analysis easier. It will be understood that cycling through multiple LEDs can be effected fast and that additional circuitry will be provided, for example for digitizing the signals obtained from the sensor, which allows the signal processing stage to process digital data; accordingly, the signal processing stage can comprise a microprocessor, data storage elements and so forth.

A variety of electrochemical (or "electroanalytical") methods measure a potential (voltage) and/or a current in a solution containing the analyte; to this end, a plurality of different techniques are commonly used. Whatever electrochemical technique is used, electrical contact must be made with the solution containing the analyte or its reaction product(s). Accordingly, where the signal is obtained by electrochemical methods, the carrier can comprise an area for electrically contacting the read-out device. The electrical contact could be established by providing electrodes in the read-out device having tips that in use come into contact with reagents in a fluid on the carrier; however, in a preferred read-out device arrangement, only electrode connection areas (that is, terminals) are provided for connecting the electrodes provided on the carrier with a voltage and/or current source, with the signal conditioning circuity asf. provided in the read-out device. Using electrodes integrated onto or into the (disposable) carrier is considered advantageous since otherwise, care must be taken remove reagents from the electrode tips after each use.

The carrier can will have an elongate form, for example with a body fluid application zone on one end of the elongate form, an intermediate connection zone comprising a microfluidic channel, and, a reaction zone and a detection zone at the end opposite to the fluid application zone.

While the electrochemical measurement itself can be effected using any of the known techniques, such as open-circuit potential potentiometry, coulometry, voltammetry,in particular cyclic or square wave voltammetry, and/or amperometry, the method clearly preferred is amperometry, in particular chronoamperometry and/or electrochemical impedance. In more detail, in potentiometry, the potential between two electrodes in a solution is determined; this may require electrodes such as a platinum reference electrode and/or a glass-membrane electrodes and hence is less preferred outside a laboratory environment.

Coulometry applies a current or potential to convert an analyte from one oxidation state to another; from the total current measured, an indication of an analyte concentration can be derived. However, problems may arise here when relying only on the absolute value of the current; thus, other methods are preferred.

In voltammetry, a potential is applied at an electrode and the resulting current is measured with a three-electrode system. The method typically requires only a small amount of analyte, making it suitable for the purposes of the present invention, in particular, since the concentrations to be determined can be low as well. It will be understood that the potential applied at the electrode may vary over time, giving rise to special forms of voltammetry such as square wave voltammetry which has in particular been found applicable for measuring glucose as analyte in body fluids such as saliva and blood.

In amperometry, a current is measured as a function of an independent variable such as time or a potential applied to the electrodes; in chronoamperometry, the current is measured at a fixed potential at different times. Chronoamperometry is typically carried out using a fixed electrode and avoiding convection or stirring. Note that voltammetry mentioned above can be considered a subclass of amperometry, where current is measured while varying the potential applied to the electrode.

It should be noted that it is possible to obtain signals simultaneously in different ways, e.g. electronically and optically and that this can help to further reduce cross dependencies or improve the precision of a measurement.

Another preferred technique is dielectric spectroscopy where the dielectric properties of a medium are measured as a function of the frequency of an alternating potential or current applied to electrodes; in this manner, additional information can be derived.

It will be obvious that where the read-out device is adapted for an electrochemical measurement, corresponding measurement means are provided, for example electrodes or electrode connection terminals, voltage and/or current sources that can apply a dc or ac signal to the electrodes, in particular an ac signal of variable frequency.

A large number of different body fluids might be used for the present invention, for example saliva, urine, sweat, tears, sperm, feces, blood or blood plasma. However, while some of these such as saliva and urine are easily available in sufficient amounts, this does not hold for fluids such as sweat or tears, the collection of which is more bothersome. Also, the amount of a given analyte might be rather low in certain of the fluids or changes in the physical analyte level are not reflected fast enough in the fluid. Therefore, urine and saliva are particularly preferred from a perspective of accessability to the fluid in practice. However, concentration of an analyte in urine may vary strongly depending on the amount a person is drinking, ambient conditions and so forth, where the variation in saliva due to such external influences can be expected to be significantly lower and hence the significance of the variability for a number of analytes can be expected to be significantly higher for saliva. Furthermore, blood- while slightly less accessible than saliva or urine given that the skin must be pierced to obtain even a single drop of blood, is particularly preferred as a large number of laboratory examinations rely on blood and hence, using a read-out device with blood allows it to be calibrated with particular ease relying on measurements of blood samples both with the read-out device and established laboratory methods. Thus, it is preferred if the group of at least two different types of body fluid comprises at least one of (blood, urine, saliva) and preferably two of (blood, urine, saliva), preferably in particular blood and saliva It will be understood that the read-out device will be suitable for use with different types of body fluid if it is adapted to read out a carrier usable with the corresponding types of body fluid.

A number of analytes can be examined using the device of the present invention given that a number of analytes of interest are present in different body fluids, in particular in at least one and typically 2 of saliva and blood. Analytes indicative of certain diseases can be for example proteins and glycosylated proteins, in particular proline-rich peptides, amylase, host-defence peptides, mucins, secretory IgA and carbonic anhydrase, proteinases, endothelin, lactoferrin, metalloproteinases, chitinase, hexosaminidase, nitric oxide radicals, fibronectin, cystatins, neopterin, cytokines, glycated proteins and fats, soluble factor CD44, telomeras, cortisol and dehydroepiandrosterone, estriol, phenylalanine, valine, and lactic acid, zinc-a-2-glycoprotein, haptoglobin, and human calprotectin). The analytes can be indicators or related to different forms of cancer such as oral cancer, pancreatic cancer, gastric cancer, infectual bacterial, viral, fungal and autoimmune diseases, endocrinology, psychiatry, nephrology, cardiology and metabolic diseases such as diabetes. It is also suggested that analytes relating to fertility periods, pregnancy, labor onset, alcohol concentration, blood glucose concentration are measured as well as indicators that may signal a need for comprehensive HIV testing. It is thus considered advantageous to offer respective different carriers that can all be used with the same read-out device.

It is also preferred if the read-out device is adapted to determine glucose or an indicator of the glucose concentration as the analyte. Glucose is an important indicator for patients having diabetes and frequent monitoring of the glucose level is necessary for diabetes patients. Accordingly, providing a read-out device capable of determining glucose in a quantitative manner is helpful for a large number of people. In this context, it should however be understood that in a particularly preferred variant, the read-out device may be usable with a variety of different carriers, each carrier usable for a different analyte such as cortisol or glucose. In particular, it is possible to code the specific analyte in a digitally readable form onto the carrier such as by using a small ROM provided on the carrier, an RFID tag, a QR tag or bar code that are read when a carrier is inserted into the read-out device. The read-out device can then load a specific program for performing an assessment in accordance with the specific analyte the carrier is adapted for or can adapt the program so as to be suitable for the specific analyte. This is particularly useful if the read-out device is usable for a variety of different analytes or more precisely with a variety of different carrier, with at least some of the different carriers usable for different analytes and at least one of the carriers being usable for detecting a given analyte in one of at least two different body fluids. It should thus be understood that where the read-out device is usable with a plurality of different analytes, it is not necessary that the carriers for every analyte are usable with two or more different types of body fluid. It will also be understood that reading out digital or otherwise coded information on a carrier is advantageous per se, that is, even if the read-out device is not adapted to read out a plurality of different types of carrier strips for a plurality of different types of analytes. Such additional information can also be used to indicate calibration information relating to a specific carrier, for example because batch-to-batch variations of the sensitivity are observed or because a carrier for one and the same analyte may intentionally be offered with different sensitivities. Such information relating to a (batch-to batch) sensitivity can be used to provide or change parameters for assessment and, where the carrier is specifically adapted to be used with a variety of different body fluids, will be helpful in particular for all or some of the different body fluids. In the context of providing sensitivity information relating to specific batches of carriers, it has already been suggested to provide every package of carrier strips with a (single) code chip carrying information relating to the overall sensitivity of the batch. This known approach could be used with the present invention as well, albeit it is preferred to include information directly on the carrier, such as by a QR code printed onto each carrier as a tag applied to the carrier after printing. Another possibility is to provide an RFID tag and to provide the read-out device with a suitable NFC interface. Then, either each batch of carriers would come with an RFID tag relating inter alia to the calibration and sensitivity or each single carrier could be provided with such an RFID tag. In a preferred embodiment, the RFID tag is automatically read out when the tag comes close to the read-out device or the reading of the RFID tag is initiated by a user. However, where a QR code is used and the read-out device is adapted to obtain the analyte-related signals optically using a camera-like arrangement, no additional interface might be needed as the QR code could be read out with the same camera arrangement used for acquiring the analyte-related signals. While reference is had to a QR code as a well established optical code having a sufficient data capacity for calibration information, other standards or even proprietary optical codes could be used as well, given that calibration information can be expected to be only several 10bytes to not more than 1 or 2 kbyte. It will be understood that information relating to the origin of the carrier might also be included; this helps to ensure that only genuine carriers from a trusted source may be used.

It is also preferred if the signal obtained is digitized and the signal processing stage is adapted to process a time series of digital values. It will be understood that the signal may be conditioned prior to digitization; for example, an electrical analog signal obtained from a photosensitive element or from an electrochemical electrode or set of electrodes may be suitably impedance matched, amplified and bandpass filtered prior to analog-to-digital-conversion.

It is also preferred if the signal processing stage is adapted to evaluate the time series locally such that an indication of the level of analyte can be output. It will be understood that local assessment /evaluation of the time series would not be necessary. For example, the time series of digitized values could be transmitted via a suitable interface to a smart phone, a smart watch, a lap top or computer or into a cloud. In case of such transmission, additional information, if procured by the read-out device, such as calibration information relating to a batch sensitivy of the carrier strip currently used, could be transmitted alongside the time series of digitized values. The transmission could be effected via any current or future suitable protocol, such as Bluetooth, Bluetooth LE, Zigbee, Wifi asf. Where the time series of digitized values is transmitted, it will be preferred to transmit an entire series of values as a packet rather than transmitting single values; this reduces the overall energy consumption and the data processing will require a minimum number of data values anyhow, so obtaining the result will not be delayed significantly. However, as the processing itself typically is neither time critical in itself (in that a response within several seconds or fractions of a second will be perfectly acceptable) and the processing load is not very high given the typically reduced number of values and calculations that need to be effected, it is preferred to provide the read-out device with a local microprocessor and/or microcontroller capable of the necessary processing for obtaining an indication of the analyte in the body fluid examined. Such indication may be an absolute concentration as is preferred in the case of glucose or an indication of the presence of an analyte above a certain threshold, which can be helpful e.g. in pregnancy tests or e.g. measurements of cortisol as a stress indicator. The local assessment is however preferred because this allows to make use of the read-out device as a stand-alone unit and avoid data privacy issues. In case a local assessment is effected, a result of the assessment can be displayed on a local display. Such local display can be implemented by a series of LEDs, an LCD or a small screen; also, an audible signal might be issued. Note that an LCD or small screen can also be used to show additional information, for example that a carrier is too old to give reliable results, that a carrier has been inserted in an incorrect orientation and so forth. It should be understood that even where a read-out device is adapted to locally determine an analyte-related value such as an indication that a certain threshold has been exceeded or that the analyte has a concentration ascertained, an interface to an external device such as a smartphone might still be helpful as this may help in keeping track of changes or in reacting to a given analyte level, e.g. by suggesting or controlling a specific insulin dosage in a diabetes patient. Accordingly, it is preferred that the read-out device has an interface to a smart device, in particular a smart phone, smart watch or a laptop.

It is preferred if the signal processing stage is adapted to process a time series comprising at least 50 samples, preferably in particular at least 100 samples, particularly preferably at least 200 samples, taken over a period of time sufficient for observing the onset of a signal increase, in particular over a period of at least 2, preferably at least 5, in particular at least 10 seconds, and/or having a resolution after digitalization of at least 8, preferably at least 10 and in particular at least 12 bit. It will be understood that too few samples will make an assessment more difficult or imprecise, in particular with preferred techniques such as chronoamperometry. In electrical impedance spectroscopy, a number of spectra over time might be acquired or a sufficiently large number of values from a plurality of different frequencies might be obtained.

As has been indicated above, the analyte level may be determined either by indicating whether the level is above or below a specific threshold or an absolute concentration of the analyte can be indicated.

As has been indicated above, it is also preferred if the interface is further adapted to read out calibration information from the carrier and the signal processing stage is adapted to determine a plurality of parameters from the time series of digital signal values and to assess the level of analyte in response to both the parameters determined and the calibration information.

In a particularly preferred embodiment, for assessing the signals, the signal processing stage is adapted to store at least two different models usable for assessing the level of the same analyte in response to the parameters from the time series of digital signal values, with each model relating to a different type of body fluid, the signal processing stage being adapted to select the appropriate model in view of the time series of values or the parameters from the time series of digital signal values respectively.

It is also preferred if the read-out device according to any of the previous claims is adapted to obtain signals from disposable carriers. These carriers may comprise reagents that react with the body fluids, in particular any analyte therein.

The invention also suggests a disposable carrier adapted for use with a read-out device as described above wherein carrier is adapted to react with an analyte in the at least two types of body fluid and interface is adapted to allow the interface to obtain signals optically and/or electrochemically, preferably by chronoamperometry and/or by electrical impedance spectroscopy. In this context, it is noted that it has been shown in the literature already that electrical impedance spectroscopy can be used to detect erythrocyte membranes with frequencies between 100kHz and 1Mhz. Since significant amounts of erythrocyte membranes are present only in blood, but not in saliva, this can be used to distinguish a blood sample from a saliva sample; while it is not absolutely necessary to know in advance whether a body fluid analyzed is blood or not, it may be considered helpful in certain instances. Hence, it is possible to carry out an electrical impedance measurement as known per se in the art to determine whether the body fluid appplied at the body fluid application zone was blood or not and both the read-out device and the carrier will be suitable adapted. It should be remarked that in an embodiment of the invention and hence in a given read-out device or at a read-out device/carrier combination according to the invention, the electrical impedance measurement could be made prior to the actual determination of the analyte in that the signal electrical impedance measurement signals are obtained before the reaction of the analyte leading e.g. to chronoamperometric signals have started. To this end, a set of separate electrodes could be placed between the body fluid application zone and the observation zone, e.g. along the microfluidic arrangement, no experimental evidence that the rather small signals that need to be applied to electrodes for an electrical impedance measurement distinguishing blood from other body fluids disturb later chronoamperometric measurements has been found yet. Identifying the body fluid as blood prior to obtaining the chronoamperometric or other analyte detection signals will shorten the period necessary for the calculation of the analyte concentration. In a more simple arrangement, it would also be possible to apply the signals necessary for electrical impedance measurement to the very same (coated) electrodes at the observation area after the chronoamperometric signals related to the detection of the analyte have been obtained. In this context, it is noted that despite any reaction for detecting the analyte in blood, in an average blood sample enough erythrocytes will remain detectable at least at the level necessary to distinguish blood from other body fluid.

In the detection zone, in a preferred embodiment, electrodes will be provided and the electrodes will be coated or be otherwise provided with a reagent such than an analyte can be electrochemically detected when electrical contact between the read-out device and the electrode (s) is made. Accordingly, some or all of the electrodes are functionalized in such a case.

The carrier can be a disposable device, in particular a cartridge, designed with an application area for body fluid, one or more detection zones and a microfluidic arrangement, in particular a microfluidic channel to contain and guide the sample to the one or more detection zones. Several detection zones can be provided in case several analytes shall be detected from the same sample of body fluid (provided the read-out device is adapted to read out corresponding signals from the several, separate zones) and/or the same analyte is to be detected using different methods such as optical and electrochemical methods. Where several detection zones are provided, the microfluidic arrangement can split body fluid applied at the application area into several parts. Being a disposable carrier, each carrier will be used one single time. While most users will not attempt to re-use a carrier, provisions could be made to prevent re-use, for example by destroying a fuse on the carrier leading to the electrodes once a measurement has been made, by placing a unique identifier on the carrier which can be read and memorized by the read-out device and so forth.

The carrier can comprise filters for the body fluid, so as to prevent froth or foam or, in particular for saliva, mucus at the entrance of a channel forming part or constituting the microfluidic arrangement to advance; as an alternative or in addition, the microfluidic arrangement itself may filter such fluid, e.g. because it is constructed with fibrous capillary material. It is noted that it is not neccessary to include in the microfluidic arrangement a separate element such as a bubble trap, in particular where a wick or the like is used to transport the fluid from the application zone to the detection zone.

The carrier may enclose the body fluid once applied, e.g. by a cover over the detection zone. This is particularly useful if the carrier is adapted for use with potentially infectious body fluids such as blood. The carrier may comprise one or more reservoirs for adding reagents to the analyte before it reaches a detection zone or may bring reagents into the contact with the body fluid only once the body fluid has reached the detection zone, e.g. in a coating on electrodes. Such reagents may be e.g. buffers or reagents that react with the analyte itself and/or reagents that block or reduce the activity of enzymes or proteins that adversely affect the detection of an analyte, e.g. by blocking proteases. The amount of reagent needed will be small in view of the typically small amounts of body fluid passing through the microfluidic arrangement and necessary for analyte sensing. Using reagents in dry form that dissolve when body fluid passes may increase the shelf life of a carrier.

Where a large volume of reagents or other fluid must be added to a sample placed on the carrier, it may become necessary to vent the detection zone and thus, a reservoir for air or an air outlet may be provided. However, as a large amount of reagents will dilute the body fluid reaching the detection zone thus reducing the analyte concentration, it is preferred - and possible- to have a carrier where no such dilution takes place. Where different reagents need to be added to the body fluid for analyte detection and/or reduction of cross-sensitivities in a specific sequence, this can be realized e.g. by passing the body fluid over areas in the microfluidic arrangement that have been impregnated or coated with the reagent; this would dissolve reagents in the body fluid. Alternatively, coating could be used that comprise the respective reagents but dissolve with a different speed. Also, one or more reagents might be present on the carrier in a dissolved form or as a liquid in one or a plurality of reservoirs that can be emptied in a predefined sequence, e.g. because the reservoirs are compressed by inserting the carrier into the read-out device in a manner ejecting fluid into a microfludic channel.

It will be understood that carriers can be and generally will be arranged such that no steps other than bringing the application zone into contact with a suitable body fluid and inserting the carrier into the read-out device need to be effected by the user and that the carrier can be (but need not be) designed such that the (empty) carrier is inserted into the read-out device prior to applying a body fluid. Also, it is possible to cover the application zone by a removable cover prior to use. The cover can e.g. be a plastic foil that is peeled off before application of the body fluid. As an alternative or in addition, the entire carrier could be placed inside a disposable envelope that is torn open prior to use. Also, it is not necessary to include in the microfluidic arrangement such functions a a bubble trap, in particular where a wick or the like is used to transport the fluid from the application zone to the detection zone.

The carrier may have a back made of or with a metal so that a temperature controlling means such as a peltier element or a heater on the read-out device will quickly alter the temperature of the carrier. Alternatively, a temperature sensor and a heater might be provided so that the carrier itself can be electrically heated to ensure a sufficiently high and/or stable temperature.

The invention will now be described in more detail with respect to the drawing. In the drawing:

Fig. 1      read-out device;
Fig. 2      carrier;
Fig. 3      ideal response curve ;
Fig 4      response curve for different concentrations of analyte /Linear ;
Fig 4b      response curve for different concentrations of analyte /log ;
Fig. 5      electrochemical reactions used for glucose detection in embodiment;
Fig 6      baseline subtracted, time shifted curves prior to normalization to unity;

Fig. 7      combination of square wave signals with a staircase potential sweep ;

Fig. 8      bad example of a feature: Signal peak height vs concentration ;

Fig. 9      scheme of training process ;

Fig. 10a      variation of parameters with concentration c of analyte ;

Fig. 10b      variation of parameters with concentration c of analyte with line of regression and error bars, same data as Fig. 10b;

Fig. 11      relation between prediction and actual concentration for test set using selected model;

Fig. 12a,b      method distinguishing different body fluids

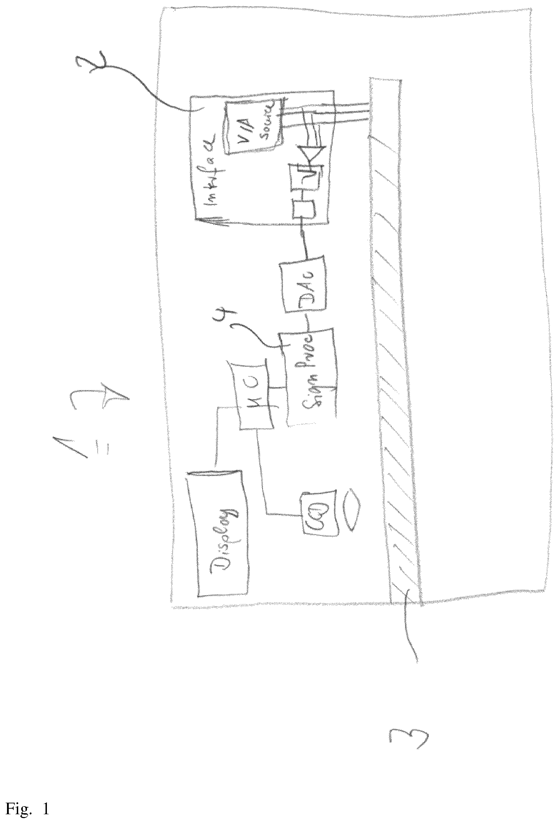

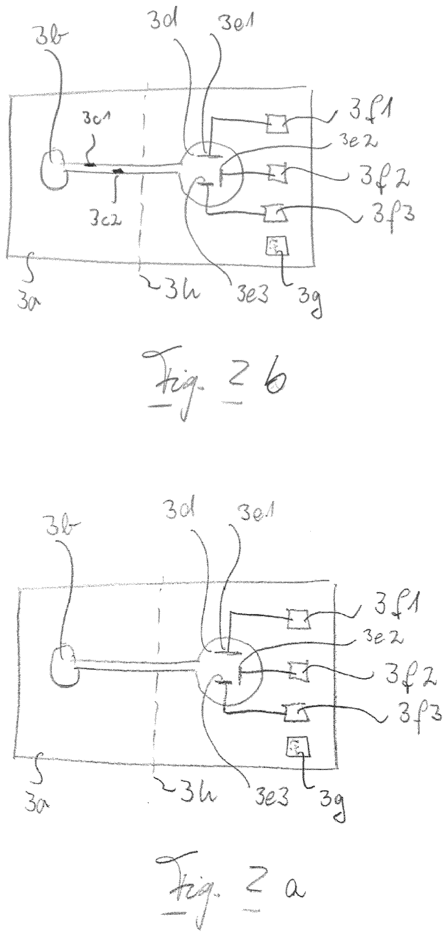

According to Fig. 1, a read-out device 1 comprises an interface 2 adapted to obtain a signal indicative of the level of an analyte in a body fluid from a carrier (reference sign 3, Fig. 2) brought into contact with one of at least two types of body fluid and a signal processing stage 4 adapted to process the signal in a manner allowing determination of the level of the analyte in a body fluid, wherein the interface 2 is adapted to obtain the signal as a time series of digitized signal strength values from a carrier which receives in use body fluid selectable from at least two different types with each type containing the analyte in a determinable amount, and the signal processing stage is adapted to assess the analyte level from the time series of signal strengths independent of the type of body fluid received on the carrier.

The carrier 3 is shown in more detail in Fig. 2 with Fig 2a showing a simple embodiment for detection of glucose in saliva and blood, whereas aspects of a more sophisticated embodiment for the protection of other analytes will be described with respect Fig. 2b. In the preferred embodiment, the carrier is of rectangular form and has a marking indicating the direction of insertion 3i.

The carrier 3 has a base plate 3a made from plastic with a rigidity sufficient to handle and insert the carrier into an entry opening of the read-out date device 1. The base plate 3a comprises a body fluid application area 3b which is connected via a recessed microfluidic channel 3c with an observation area 3d, where electrodes 3e are provided; in Fig. 2, a set of electrodes 3e1, 3e2, 3e3 is shown. In the embodiment shown, three electrocdes are provided as is common for a number of electrochemical analytical methods, but depending on the exact method, more or less than three electrodes could be provided. Each of the electrodes 3e is electrically connected to a contact pad 3f on the upper surface of the carrier 3, so that in the embodiment shown, three contact pads 3f 1,3f2 and 3f3 are provided. In the embodiment shown, the electrodes have a gold surface.

In the embodiment used for glucose detection shown in Fig. 2a, the microfluidic channel is dimensioned such that body fluid applied at the body fluid application zone is transported via capillary forces to the observation zone 3d. Due to this transport, a precise volume is filled into the reaction chamber of the carrier upon contact with a body fluid sample due to the capillary action of the microfluidic channel; all necessary reagents, namely a suitable enzyme and a mediator, will be provided by coatings on the electrodes. As in the embodiment shown, the carrier strips are used for glucose sensing, GDH-FAD enzyme and ferricyanide mediator reagent, are deposited on the gold surface of the electrodes. Accordingly, the electrodes are surface functionalized for analyte detection.

However, in a different, more sophisticated embodiment, as shown in Fig. 2b, the recessed microfluidic channel is filled with a fibrous material; in that case, any body fluid applied at the body fluid application area 3b would also be transported via capillary forces to the observation zone 3d. Along the path of the microfluidic channel, the fibrous material can be impregnated with additional reagents dissolvable in all or some of the body fluids with which the carrier is to be used. The amount of reagents is such that in the initial bolus of fluid drawn via capillary forces through the microfluidic channel, a sufficient amount of reagents is dissolved. The areas where the fibrous material is impregnated are designated as 3c1 and 3c2 in Fig. 2 to indicate that when necessary, a plurality of areas may be provided and, as is possible, these areas are spatially separated so that any reaction between the body fluid or components therein and the reagents in the respective areas 3c1 and 3c2 can be effected in a sequence and/or with the timing necessary for the detection of a specific analyte and/or use with specific body fluids. However, in some cases, it might be possible and preferred to impregnate the fibrous material in one area with more than one reagent. It would also be possible to impregnate the fibrous material with one or more reagents needed for only one of several different body fluids; dependent on whether the reagent might tamper with the detection of the analyte in other body fluids, care could be taken to use a reagent that cannot be dissolved easily in other body fluids, for example because of the different pH of different body fluids. Also, it is possible to not impregnate any area along the microfluidic channel at all; in the case of glucose detection, it is possible to only code the electrodes so that a reaction starts only once the body fluid has passed through the microfluidic channel 3c and has reached the electrodes 3e.

Furthermore, this holding for the embodiments in both Fig. 2a and Fig. 2b, each carrier 3 comprises a calibration information area 3g where information relating to the calibration of the production batch has been stored in a manner readable by the read-out device. In the embodiment shown in Fig. 2, the calibration information area comprises an optically detectable QR tag with all relevant calibration information as well as information relating to the analyte-in the embodiment shown glucose-and additional information such as a batch number, a producer of the carrier and so forth.

The microfluidic channel, the fibrous material therein if any, the observation zone and the electrical connections leading to the connection pads 3f1 - 3f3 are protected by a thin, water-insoluble coating; the electrode contact pads 3f1 -3f3 are not coated so that they can be contacted electrically in the read-out device.

In a preferred embodiment, it is possible to fold the ends of the carrier onto each other such that no contact with body fluids of the received use thereof is made when disposing of the used carrier. To this end, the carrier end with the body fluid application zone is foldable onto the end where the observation zone is provided, as symbolized by a folding line 3h; a corresponding perforation of the base plate may be provided. Means are provided to hold the folded carrier together, such as a self-adhesive area, flaps or the like (not shown).

Describing now the read-out device 1 in more detail, the read-out device has a funnel-shaped opening for inserting the carrier in a predefined orientation. The opening is deep enough to insert the carrier so far that the electrode pads come into contact with corresponding electrical connection terminals of the read-out device when the carrier is fully inserted. When fully inserted, the body fluid application zone will stay outside of the reading device and will be at a spatial distance from the opening such that the opening will not be contaminated by body fluid spilled in the direct vicinity of the body fluid application zone or in case a user has or decides to only apply body fluid only once the carrier has been inserted.

On the inside of the read-out device 1 and close to the entrance opening, an optical sensor, in the present application a CCD sensor with a lens in front thereof having a focal plane at a distance where in use the QR tag of the carrier passes underneath is provided that is adapted to detect insertion of a carrier into the opening and to read out the QR tag provided on the carrier.

In the observation area, the read-out device 1 furthermore comprises a number of electrode input terminals which are adapted to electrically connect to the contact pads of the carrier which in turn are connected to the electrodes of the carrier, so that once the carrier is fully inserted into the read-out device, an electrical connection between the read-out device and the electrodes exists. These input terminals are part of the interface 4 and are connected to a voltage/current application stage comprising a voltage/current source for alternating and direct current as used in chronoamperometry, but in other embodiments could comprise a voltage/current source adapted for different methods of measuring, such as electrical impedance spectroscopy; for multianalyte use of the reader the source will be correspondingly programmed. Furthermore, suitable circuitry is provided to obtain and condition an analog signal when a bolus of body fluid is brought into contact with electrodes . The signal conditioning comprises stages such as an amplification stage, a bandpass filter and an impedance matching stage and is connected at its output to an analogto -digital-converter which in the embodiment shown is a 12 bit analog-to- digital converter capable of providing 12 bit digitized values with at least 100 khz bandwidth.

The stream of digitized values is sent to a digital signal processing stage adapted to analyze it in a manner suitable for assessing the amount of analyte in each of the body fluids. To this end, in the preferred embodiment described, the digital signal processing stage is adapted to identify the body fluid applied to the body fluid application area from the time series of values and to then determine the analyte in response to both the time series and the identified body fluid. Accordingly, in the preferred embodiment, the digital processing stage is adapted for a two-step assessment, the first step comprising a body fluid type identification and the second step comprising an analyte amount determination step, both steps relying on a time series of digital values and/or features (parameters) derived therefrom. It will be understood that to this end, the digital processing stage will comprise a memory for storing processing instructions and/or operations for digitally automatically process the time series of signal values in an automated, computerized way.

In the preferred embodiment shown, an illumination arrangement comprising a plurality of LEDs of different colors and an optical detection arrangement comprising a plurality of light sensors of different spectral sensitivity is provided in addition to the electrode contacts such that the observation area can be illuminated with light of different colors from the LEDs and light coming from the observation area on illumination can be detected with the light sensors of different spectral sensitivity. In a preferred embodiment, LEDs are used that emit blue, red, green and yellow light of an appropriate wavelength range and the light sensors will have a spectral sensitivity such that fluorescent light having a wavelength shorter than that of a respective illumination wavelength can be detected. It is explicitly noted that such LEDs can be combined in common into one single LED component (such as a white light LED that can be appropriately excited so as to emit the respectice color) or that single LEDs can be used that are equipped in particular with separate spectral filters. Using spectral filters is preferred as this allows to more precisely specify a wavelength. Also, some or each of the sensors can be provided with a suitable spectral filter. Where a plurality of different analytes is to be measured optically, one or more different sensors can be provided for each analyte or a group of analytes. It is noted that for the purpose of glucose detection, such optical sensors will not be used.

A further set of LEDs is provided on the outside of the read-out device to signal to the user that a carrier has been inserted, that a measurement or evaluation is ongoing, that a result has been obtained or that an error has been occurred. An error can be signaled for example if some time after insertion of a carrier no signal at all can be obtained, if the signal is considered insufficient or incomplete, e.g. because the user has waited a long time between applying a saliva sample on the application zone of the carrier and insertion of the carrier into the read-out device, because the signal obtained strongly deviates from what is needed for a useful evaluation (as will be explained hereinbelow) or e.g. because the battery is low. It is to be mentioned that instead of or in addition to using a set of LEDs, acoustical information could be given or a display such as an LCD or OLED matrix display could be activated, but using and controlling a LEDs is simple, saves energy and can thus be preferred. However, the read-out device in the embodiment shown also comprises a numeric or alphanumeric display that is adapted to display to the user the value of an analyte concentration in a sample just analyzed.

The read-out device furthermore comprises a micro-controller that controls the operation of the different stages and is in particular capable of decoding the signals obtained in particular from the optical sensor close to the entrance for reading out the QR tags. The micro-controller furthermore is adapted to start a timer upon insertion of the carrier into the opening, to sense when the carrier has been fully inserted into the reader and to start the voltage/current application stage for applying a voltage or current to the electrodes; the micro-controller further is adapted to switch on the signal conditioning stage and digital-analog converter upon the beginning of the insertion of the carrier into the reader as sensed by the optical sensor close to the entrance and to switch off the read-out device after a given time period which may depend on the QR tag on the carrier so as to account for different times needed for different analytes to detect a useful set of values. The micro controller furthermore is adapted to store and process the digitized values and to output results of the analysis on the local display and/or via an interface (not shown) to a device such as a smartphone, a smartwatch, an insulin pump or the like. If any direct connection to a medical device such as an insulin pump is provided, the transmission of any data might be encrypted so that no third party can interfere with the transmission or fake control signals.

The operation of the read-out device will now be described.

The read-out device is basically used in that a user places a minute amount of body fluid, in the present embodiment either blood or saliva, on the body fluid application zone of a previously unused carrier and immediately thereafter inserts the carrier into the opening of the read-out device until the contact pads of the carrier come in electrical contact with the electrode input terminals of the read-out device.

When inserting the carrier into the read-out device, the QR tag of the carrier passes underneath the lens of the CCD sensor and is read out. On sensing the QR tag, a timer is triggered by the micro controller; the QR tag is decoded and all relevant calibration information as well as information relating to the analyte is decoded from the QR tag and hence the specific signals that need to be generated by the voltage/current stage can be determined. In the current embodiment, it is determined that a user is to determine glucose in either blood or saliva.

The calibration information, in particular calibration coefficients encoded in the QR code are stored as current values for the subsequent assessment of signals to be obtained in a volatile coefficient memory. (Note that in other embodiments, calibration parameters could be read in separately from use, for example from a QR code or RFID pack provided with each package of carriers.). Then, the voltage/current stage is controlled so as to apply the correct signals at the electrode input terminals once contact has been made between the contact pads of the carrier and the electrode input terminals.

Once the carrier has been inserted fully into the read-out device, electrical contact is made between the electrode pads of the carrier and the input terminals of the read-out device . At the same time, the standard signal acquisition process including conditioning and digitizing of signals at the electrode input terminals is started with a suitable frame rate as used later on. Accordingly, from the beginning of insertion of the carrier into the read-out device 1, the signals from the input terminals will be conditioned and digitized to create a time series of digitized signals. These signals are stored as a time series of values in local memory accessible to the micro controller.

Until such contact has been made, the timer is periodically read out and compared with a pre-set first threshold by the micro-controller. If the first threshold is exceeded, a warning is issued to the user indicating that the user might have erroneously inserted the carrier not far enough into the read-out device. Once the warning has issued and until contact is made between the electrode pads of the carrier and the input terminals of the read-out device, the timer continues to be read out periodically and the timing is compared with a pre-set second threshold by the micro-controller. If such second threshold is exceeded as well without contact being made, the read-out device will be switched off to save batteries.

However, in a typical case, the user will immediately fully insert the carrier and this will be detected by the micro controller in the read-out device or will at least insert the carrier fully within a time limit set. After contact between the electrode pads of the carrier and the input terminals of the read-out device is made, the microcontroller will obtain values from the analog- to- digital converter and store them in a volatile memory for processing once the time series has been recorded for a sufficiently long time.

From this time series, the analyte concentration is then determined in a manner such that use of either of the two body fluids saliva and blood will yield the same results.

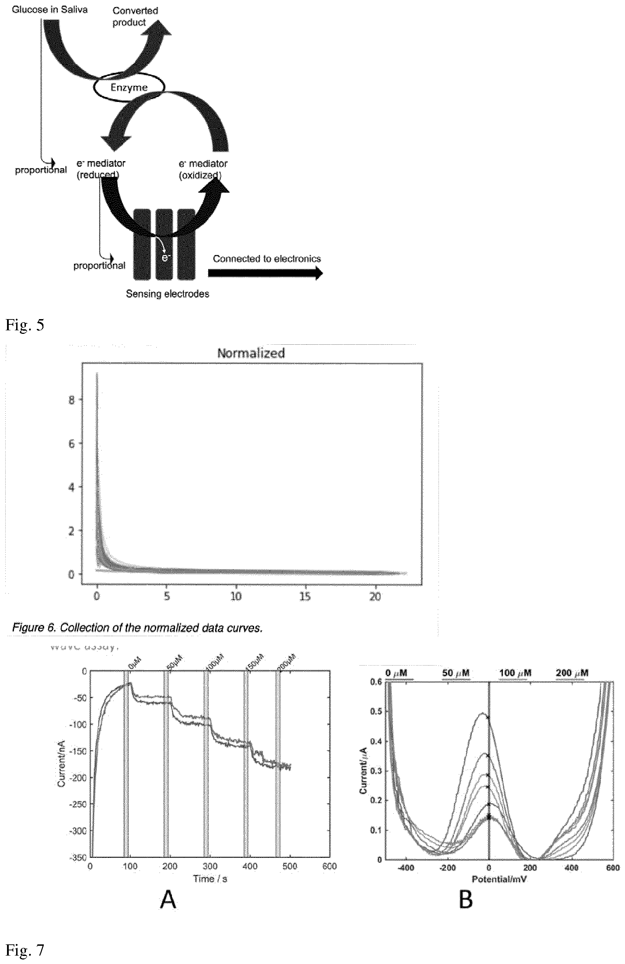

In order to show how an analyte concentration can be determined from the time series in a precise and fast manner despite allowing use of different types of body fluid, a more detailed explanation of the electrochemical analysis steps involved in the specific embodiment and the processing of the signals obtained from the reaction will be given hereinafter with respect first to typical examples of the signal curve detectable at the electrodes. The theoretical chemical processes of electrochemical detection of glucoses used in the present embodiment are shown in Fig. 5.

As indicated, in the present embodiment, GDH-FAD as an enzyme for glucose sensing is employed together with a mediator, namely a ferricyanide mediator. The enzyme as well as a ferricyanide mediator reagent are deposited on electrodes with a gold surface. Fig. 5 shows that the sensor chemistry used in the present embodimdent relies on the electrochemical signal transduction of the glucose concentration information into the measurable electrical signal. This transduction mechanism involves a two step process; in the first step, glucose present in the body fluid is oxidized by glucose selective enzyme. This oxidation process reduces electron mediator molecules. The mediator molecules can then transfer electrons to the electrode surface in a process easy relatively easily compared with the bare enzymatic reaction., with . In the second step, the electron mediator molecules are oxidized by probing potential scans applied by the read-out device to the electrode surface. Thus, the mediator oxidation process generates an electrical signal in form of charges (electrons) collected by electrodes over time. The corresponding electrical signal can then be correlated with the glucose concentration present in the given test sample.

When the enzyme reacts with glucose and generates electrons which then drive the redox reaction on the surface, the reaction is probed by the read-out device by applying voltage sequences. In the embodiment shown, ouput of a combination of square wave signals with a staircase potential sweep as shown in Fig. 7 is used and the time series of low current signals coming from the carrier strip using glucose oxidase and Prussian Blue based redox chemistry during the potential sweep are recorded as digitized values and stored in the memory by the micro-controller.

It will be understood that when the carrier electrode pads get into electrical contact with the input terminals of the read-out device, no body fluid will have reached the electrodes yet, given that inserting the carrier into the read-out device has been checked to be sufficiently swift. Accordingly, directly after such electrical contact has been made, the signal values at the beginning of a time series will still correspond to a base line. Then, body fluid will reach the electrodes that already are in contact with the input terminals of the read-out device and the values of the time series will show, so that after a number of values representative for the base line, a distinct signal increase in response to the reaction(s) between analyte (or analyte related components in the body fluid) and reagents can be observed.

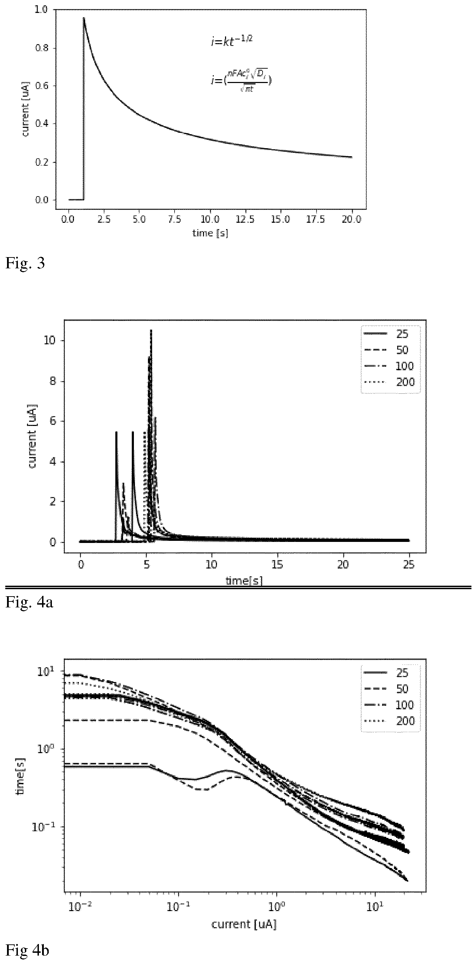

In more detail, in the specific embodiment described here uses chronoamperometry and is thus an electrochemical assay. A theoretical response curve of an electrochemical assay is shown in Fig. 3, showing the signal during a period preceding the onset of the detected reactions), at the beginning of the reaction(s) and during the subsequent (decay or decrease) period. If the body fluid sample reaches the electrodes only after the carrier has been fully inserted, this is roughly the standard pattern to be observed. If the carrier is inserted too slow, there will be no initial base line period and accordingly, it can be determined that in such samples, relevant information is missing, the entire time series can be discarded and an error message can be issued (note that in certain instances, a value could still be determined but with a significantly lower precision, which would be acceptable only for certain, non-critical applications). In the disclosed and preferred example, a minimum length of baseline observations such as .5 seconds is observed as this allows to determine a zero background signal due to the electronic circuitries involved.

From Fig. 3, it can be seen that initially, the change in the signal is strong in that a steep signal step is observed when the body fluid reaches reaches the electrodes. This can easily be detected in the time series of digitized signals in that at first, a rather steep increase of the signal values must be observable. In contrast, a rare case may occur where no signal increase at all is observed - for example because no body fluid at all has been applied prior to insertion of the carrier into the read-out device or because the amount of body fluid had been so small that it was not sufficient for a bolus to reach the electrodes. In both cases, a warning can be issued and the assessment can be stopped. It will be understood that in other embodiments without acquiring signals immediately after electrical contact is made between contact pads and input terminals. a situation might occur where a fluid bolus has reached the electrodes prior to the moment where the electrodes make contact with the input terminals. In such a case, no prior zero signal is observed and the signal immediately starts to decay. As this prevents the determination of an actual peak value, such situation must be identified and preferably prevented; therefore, use of a timer is helpful. In the present embodiment, triggering a timer for measuring time from first insertion to full contact between electrodes and inputs helps to prevent such situations.

In the standard case, where sufficient body fluid has been applied, the steep change in signal will be observed in the digitized signals obtained and with a sufficiently high sampling rate, the initial steep step response might extend over several sampled values; however, thereafter the signal will decay over time and the decay is affected by factors such as the (body fluid and patient dependent) diffusion coefficient. While the onset of the signal (that is the steep increase after a zero line signal) is observed and for some time thereafter, the signal values continue to be sampled, preferably with a sample rate of app. 1khz (although in other embodiments, the sample rate could be higher such as e.g. 2 kHz, 3 kHz or up to 10khZ without significantly increasing the need for more processing power. ) As will be understood, after some time, the signal will decay because more and more of the analyte in the body fluid close to the electrodes has reacted.

In an ideal case, that is, where a large reservoir of reagents is available around the electrodes and only the reaction to be observed is relevant (meaning in particular, that no cross-sensitivies and the like are relevant), the behavior of the signal is controlled by diffusion. Under such idealized conditions the signal will follow so-called Cottrell equation like dynamics with respect to the decay of the curve which obeys the following formula:

$$i = \text{const} / \text{sqrt}(t)$$

where

$$\text{const} = n*F*A*c^o_j * \text{sqrt}( Dj / pi )$$

with

i = current, in units of A
t = time in s.
sqrt= Square root
pi= 3,14....
n= number of electrons to reduce or oxidize one molecule of analyte
F = Faraday constant
A = area of the (planar) electrode in cm2
$c^o_j$ = initial concentration of the reducible analyte in mol/cm3;
Dj = diffusion coefficient for species in cm2/s

For a laboratory test tube experiment, where the chemical reaction related to the current is diffusion controlled, it can be seen that the maximum current will only be related to the analyte concentration and the curve observed in a laboratory experiment will corresponds to the above formula very closely, but it will be understood that in practice using a small carrier and minute amounts of body fluid, deviations from such ideal behavior will occur. Nonetheless, the signal obtained might follow by and large the formula above. Accordingly, the observed behavior for a carrier will be somewhat different, for example in a setting as in the present embodiment where transport effects on a carrier are relevant or where the analyte reacts with a reagents that may have a reactivity varying sufficiently strong, deviations occur.

So, while it can be understood from the formula indicated above, and seen in Fig. 2 and also from Fig. 3 showing variations of typical curves, that an initial decay will be observed and that this initial decay be quite fast in typical typical case, there will be deviations from the ideal behavior.

Given the temporal behavior of the curve, it is useful to have a sufficiently high sample rate so as to reduce interpolation errors. With a sufficiently high sampling rate, the initial steep step response might extend over several sampled values; while the onset of the signal (that is the steep increase after a base line signal) is observed and for the time thereafter for which signal values continue to be sampled, a sample rate of app. 1khz can be selected (although in other embodiments, as stated before, the sample rate could be higher such as e.g. 2 kHz, 3 kHz or up to 10khZ without significantly increasing the need for more processing power. ) However, the signal values might also be sampled with a lower sample rate in some cases, e.g. 10 or 20 Hz, e.g. if the relevant chemical reactions between analyte and reagents take place over a period of several seconds or several ten seconds- in such case, the resolution of the signal should however be sufficient to at least correctly describe the "tail" of the signal following the peak. It will be understood that sampling with a sample rate slightly higher than that expected to be necessary in view of the chemical reaction rates and the requirement to have a sufficient number of signal values in a time series (such as preferably 50, 100, 200, 300, 400, 500 or more samples) over a sufficiently long observation period (such as 1 sec, 2,sec, 3sec, 4,sec, 5 see up to e.g. 10 secs) allows to average over adjacent values which might help to reduce noise. Note that generally it is preferred to have observation times of no more than 15 secs to 60 secs as otherwise, the readout device might be considered to be unfavorably slow by users. In particular but not only where more time is to elapse during a measurement, an indication could be given to the user showing that the measurement or the evaluation of data still goes on, e.g. by a blinking LED with the blinking period indicating the remaining time of measurements, or by counting down measurement time on a display such as an LCD display.

A sufficiently high sampling rate is also useful to analyze the behavior of the signal "tail". It has been found that this signal tail gives valuable information, so a good resolution both with respect to time and to amplitude is helpful in determining an analyte concentration. A sufficiently high sample rate also helps as to reduce errors due to interpolation otherwise necessary.

As indicated above, using the chemical process indicated above, in an ideal case a signal will be obtained as shown in Fig. 3, with a signal strength given by the above formula. However, in practice, deviations from the theoretical values over time will be observed with respect to the signal form in Fig. 3 and the overall signal strengths. Now, such differences between practice and ideal case may arise e.g. because constituents of the body fluid might adversely affect the reactions or to the contrary promote the reactions, because the activity of enzymes used in the carrier will vary or because additional body fluid will reach the electrodes after the onset of the reaction. Such effects will vary from sample to sample and even from carrier batch to carrier batch. In practice, this leads to significant deviations from theoretical ideal behavior- and these deviations can be taken into account to obtain a good measure of analyte concentration.

The variations - and thus deviations from the ideal curve- can be seen in Fig 4 where for each of a number of different analyte concentrations, response curves obtained with a plurality of different carriers are shown. In more detail, Fig. 4 shows the signal values obtained over time for 4 different concentrations of glucose in artificial saliva, with 3 samples shown per concentration; these curves have been recorded in a laboratory environement using strips with GDH-FAD as enzyme for glucose sensing (together with standard laboratory data acquisition equipment rather than the read-out device of the invention) was employed. Despite using laboratory data acquisition equipment, the signals and data acquisition parameters closely correspond to that of an actual read-out device.

In more detail, in Fig. 4, measurements for different concentrations of analytes have been plotted in different ways depending on the concentration, namely as solid line, as a dashed line, as a dash-dotted line and as a dotted line. Overall observation time after the initial step response is no longer than about 22 secs for any of the curves. After that time, the signal can be seen to have significantly decayed for all samples.

As can also be seen in Fig. 4, the signal peak and onset thereof occurs at different times. This is irrelevant for the embodiment shown as it can be attributed to variation in starting data acquisition in the laboratory setting; while a similar observation would be made with a carrier in a read-out device - as the time until the onset of the step is basically related to the time between end of insertion and the time the fluid reaches the body fluid then reaches the electrode thereafter, this would also be irrelevant for the present embodiment. Note that in other embodiments care would be taken to also detect the time body fluid is applied to the carrier or passes a specific part of the microfluidic arrangement- in such a case, information could be derived relating to the time the body fluid needs to pass thru the microfluidic arrangement so that in such other embodiments, information relating e.g. to a viscosity could be derived.

However, what Fig. 4 also shows - and this is relevant for the embodiment described as well-is that for some measurements, the initial signal peak strength for a low analyte concentration can be higher than the initial signal peak strength obtained for a high analyte concentration. Also, one and the same analyte concentration may lead to significantly different signal peak strengths. From this, it can be concluded that the signal peak strength in itself or alone is not a good measure for analyte concentration determination and that accordingly, a measurement relying only on the strength of a signal peak at the onset of detection will not be very precise.

Nonetheless, despite the decay of the signal, surprisingly valuable information can be derived from the way the signal decays; it is possible to derive a rather precise measure of the analyte concentration if not from just the signal strength at the peak, but from the entire time series including large parts of the signal decay are considered. While no significant difference in the behavior of values in the signal tail can be easily deduced in a linear plot, in Fig. 4b, a logarithmic plot is given and there, variations from curve to curve and hence sample to sample, can be seen more clearly than in the linear plot. Note that Fig. 4a and 4b are showing the exact same data. As can be seen, differences between curves are clearly distinguishable. Nonetheless, there still is significant overlap of curves even in this graph, particularly in the tails of the graph. Surprisingly, it still is possible to derive from the curves a good measure of an analyte concentration.

In order to determine an analyte concentration from (non normalized to unity) time series, the most simple way a person might use would relate the peak current signal value as a specific voltage range seems to be associated with the glucose concentration present in a sample-however, as indicated above, this would not be sufficient and the form of the signal tail should at least also be referred to in order to obtain a more precise value of the analyte concentration.

In the context of the present embodiment, the tail of the signal will be understood to relate to those values that here are at least 0,1% lower than the maximum in the series of digital values obtained for any given carrier. Other embodiments might use values only if they are at least 0,5%; 1%, 2% or 5% or anly value inbetween lower than the absolute maximum value or use the entire curve including the maximum. In a simple implementation, the highest digital value obtained during data acquisition can be used as "maximum"; this will give a very good estimate of the actual peak value particularly if the sampling rate is high. In a more sophisticated embodiment as presently described, the maximum can be calcuated using e.g. spline techniques and/or an attempt can be made to fit an initial part (with samples within the two or three times the period needed for the curve to rise to the maximum defining an "initial part"-note that the end of such initial part may overlap with the tail) to an ideal curve e.g. according to the formula indicated above or e.g. to an average curve obtained with a plurality of carriers of different batches or a plurality of carriers from the same batch during calibration. In this way, a best value for a true maximum can be referred to even though sampling of the analog signal has been such that due to the sampling the highest digitized value is slightly lower than the highest analog value. Furthermore, a signal tail may also be construed to relate to ALL values smaller and obtained after the peak value, to all values smaller and obtained later than 0,1%, 0,10%, 1%, 2%, 3%,4% or 5% or any value inbetween 0,1%-5% and obtained after the peak value. In general, it will be preferred to include more values, hence a lower percentage is preferred - nonetheless, situations might occur where the higher values are more strongly influenced by transport effects such as additional body fluid still reaching the observation area while later (and thus lower) values are influenced more strongly by wanted and unwanted chemical reactions. Note that it it also possible to include the peak, analyzing the transient time behaviour of the entire curve. Where not the entire curve is used, corresponding information relating to a percentage for a given batch and/or analyte might be included in the QR tag.

As stated above, in order to become more independent of the maximum signal value, in the preferred embodiment, the curve obtained for a given sample will be normalized. To that effect, the point in time where the signal has reached the (interpolated or observed) maximum is considered to be the zero point in time so that the peaks of all curves and thus the decay thereof are starting at the same point.

Furthermore, the baseline is to be subtracted from the curves time-shifted in this manner. As baseline, the mean (positive) value that was observed prior to body fluid reaching the observation area is understood. As the embodiment described requires that the base line will have a length of at least 0,5 secs, this is easily possible.

When determining the base line value to be subtracted from the signal, care can be taken not to include values from the rise of the signal- given a sufficiently high sampling rate, this can be done by relating only to samples that have been obtained at least a specific number of samples such as 10,20, 30, 50 or 100 samples before the peak. The exact number will of course depend on the sampling rate and the typically observed signal rise time. The result of time shifting and base line subtraction is shown in Fig. 6. Following the time shifting of the signals and the subtraction of the base line from the signals, all curves will have the peak at virtually the same time and the long term limes will be zero for all signals. As the curves will still have a different maximum peak value, a normalization such that peak value= 1 (or any other value such as e.g. 512, 1024, 2048 or 10, 100, 1000) is effected. In this manner, the form of the curve can now be compared and information can be derived more easily.

The advantage of the normalized curves is that now, patterns which are affected by various interferences can more easily be considered; in particular, the characteristic shape of the signal will be affected by any influence on the redox signal transfer (e.g antioxidant molecules such as ascorbic acid) as well as diffusion processes. This is particularly true for rather low levels of glucose concentration, e.g. as there, signals due to cross-sensitivities will be of similar size as the signals actually sought. Therefore, the method is particularly for determining the low concentrations otypically observed in saliva.

Thus, in the present embodiment, instead of focusing on only the peak current signal, the overall signal pattern can be considered. For this, the determination of a concentration or concentration range or presence of an analyte can simply rely on specific features of the time series of signals so as to reduce the influence of or filter out completely anomalies or interference signals that are caused e.g. by cross-sensitivities to specific chemicals present in a given sample.

In a more simple form, parameters relating to specific features could be calculated and then a linear correlation equation with linear coefficients for the respective features could be used to calculate an overall analyte concentration. person will understand that a selection among a plurality of parameters can be made to obtain a good linear approximation in this manner.

It is possible to consider that the current signal from the sensor strip actually is quite complex. For that, machine learning methods can be applied to take into account similarities and differences between curves obtained under different conditions, but with similar concentrations of analytes.

Accordingly, the micro-controller of the read-out device in a preferred embodiment will be able to apply such methods or to at least transfer the time series of values to a device such as a smart phone having quite powerful data processing capabilities. This can e.g. be done using a low power data transmission, although it can be understood that despite the fact that the processing power of the read-out device will typically be limited, it still is possible to process the time series in the read-out device, as the length thereof is limited - even if a high sampling rate such as 100Hz or 1khz is used, as only about 20000 values need to be considered given the time until the signal has decayed to a level that can hardly be distinguished from noise in view of the resolution readily available with analog-to-digital-converters suitable for low-powered portable devices using batteries such as the read- out device.

To this end, the best features to determine an analyte concentration can be selected during a calibration of a variety of carrier strips from a variety of batches using one of several selection techniques. In one experimental set up, more than 20 different features of the normalized curves have been determined and examined with respect to their relevance to the analyte concentration. These features include e.g. the mean value around the peak averaged over 10 seconds, variations on the subject of autocorrelation, the temporal characteristics of the peaks, and the width of the peak. For the embodiment described here for analysis of glucose in saliva and blood, the following set of parameters has been found to be particularly relevant: the area under the curve, the value of the autocorrelation function at t=10s, the reduction of the current after 1s and the reduction of the current after 5s. Some of these features are shown in Fig. 7.

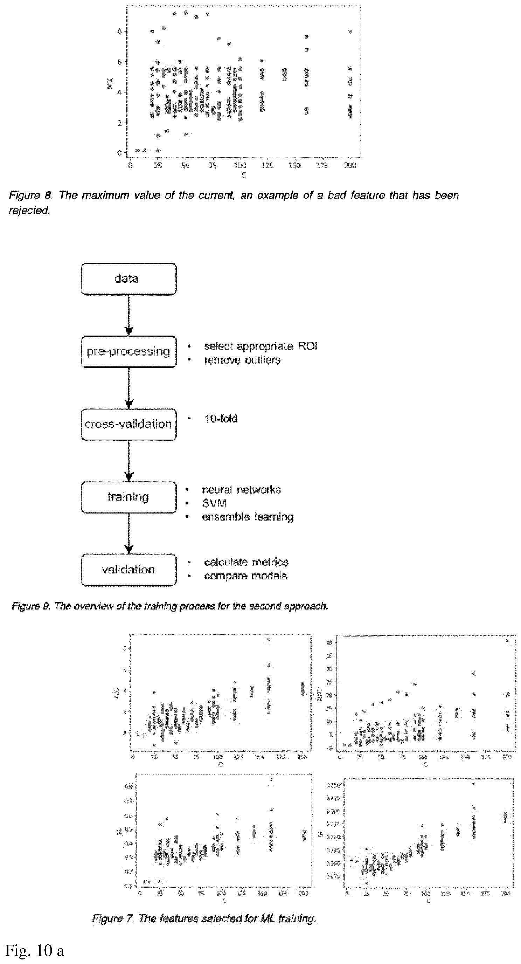

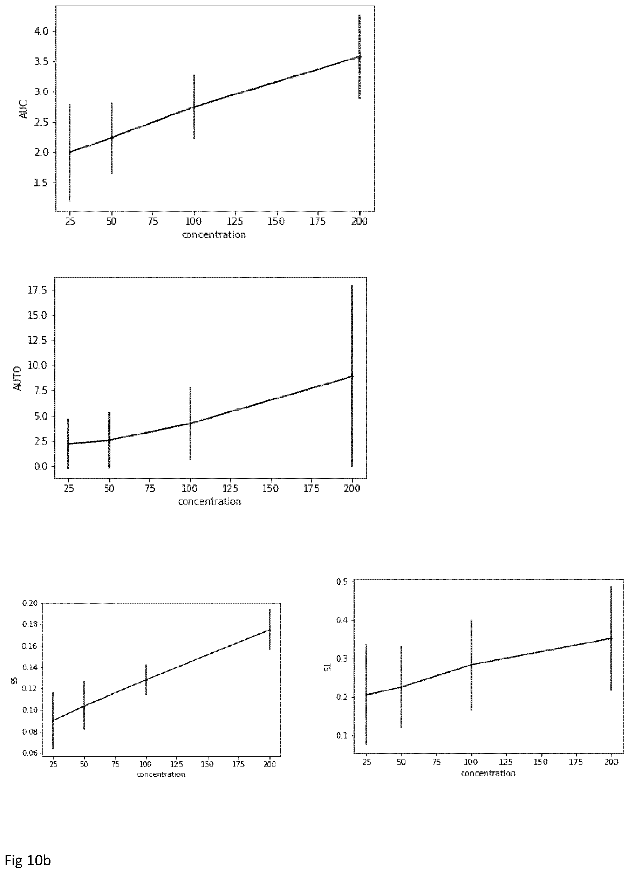

In more detail, in Fig. 10a, the variation of such parameters with concentration c of the analyte is shown for a large number of different samples at each of a number of different known concentrations. It can be seen that for any given fixed concentration selected, basically all parameters show some variations from sample to sample; still, it can also be recognized, that in some of the parameters depicted, a change in concentration will lead to a rather notable change in the parameter distribution, leading e.g. to a general increase of values of the parameter "S5" with concentration (parameter S5 being the reduction of current values 5secs after the maximum of a peak in a curve has occurred). This can also be seen in a similar Fig. 10b, where instead of plotting the single samples, a regression curve has been calculated, with the error bars from the single values shown for some of the concentrations considered. In contrast, the maximum value of the current (as given prior to normalization of curves to a peak height of unity) is not correlated with the concentration as clearly as would be expected, comp. Fig. 8.

As stated above, a number of such parameters can and should be selected for determination of the analyte concentration.

Now, a number of possibilities how to make such a selection exist, for example multivariate regression analysis where from a large number of parameters determined for each time series, some are selected "by hand", that is by simply assuming that they might be more important than others and multilinear coefficients can then be determined using standard statistical methods. These multilinear coefficients determined in calibration measurement can later be used later on during an actual measurement of an analyte by calculating the same set of parameters for features found to be relevant during calibration and combining the parameters with the multilinear coefficients calculated from the calibration measurements. Accordingly, in mass production , multilinear coefficients can be determined in calibration measurements for each new batch of carriers and the QR tags provided with each package of carriers from these batches will then encode the multilinear coefficients. Note that it would be even possible to determine different features for each batch and to encode the features most favorable for a given batch into a QR tag or similar data information memory as well.

A more preferred possibility currently favored by the applicant is to establish a model without rather arbitrary feature extraction, that is without arbitrarily choosing suitable parameters. To this end, the raw data time series can be used in a training process as shown in Fig. 9. Accordingly, in a first pre-training step, a region of interest (ROI) is defined. It has been found that all signal values that are measured within the five second after a peak should be used for further analysis and processing; this is a reasonable choice as can be seen in the respective figures of the curves, showing that early after the peak, the signal is still rather large and thus less affected by noise. Following the step of defining a region of interest, for obtaining a training set, curves are eliminated that can be considered outliers. Then, all remaining samples with appropriate ROI are used in a dataset for suggesting models as obtained by different training techniques such as neural networks (NN), support vector machines (SVM), and an ensemble model. For NN, a multilayer perceptron (MLP) and long short-term memory (LSTM) were tested, and for ensemble learning, random forest was selected.

Thereafter, once a plurality of different techniques has been used to suggest useful models, the most suitable model has to be selected, that is the model yielding the best results in determining an analyte concentration from a time series.

Generally, a model can be obtained by any of the techniques using AI training techniques. For this, a set of known data is split into a training part and a validation part; the training part is then used to train a model with the specific technique and once a model has been established by such training, it is used to calculate values from the remainder of the set, that is the data of the validation part. If the values obtained with the data of the validation part correspond closely to the (known) real values, the quality of the model is good, otherwise the quality is bad. As a precise definition of "good model" or "bad model" is needed, a key metric must be established that allows to compare all the models obtained by the different techniques.

In the present case, this can be done as follows: First, the entire data set is randomly split in 10 equally sized parts. Each part is used as a validation part for every technique considered, while the respective remainders of the data set are respectively used for training models. This will thus allow to train ten models for each technique.

For this, with any given technique and every data set of the ten splits, first, a model is trained with the training set of the respective split, yielding regressors. Then, these regressors are used with the validation data of the respective split to determine the alleged analyte concentrations. As the actual analyte concentrations for all samples are known, it is possible to compare the analyte concentrations calculated with the regressors obtained to the (known) actual values of the analyte concentrations. Accordingly for any given training technique, an absolute error and an absolute relative error of the calculation can be established for all 10 models relating to a different one of the 10 different splits. This in turn allows to calculate a mean absolute error and a mean absolute relative error for every model trained with a given 1:9 data split. Considering all (ten) models obtained for each technique, even a standard deviations of the a mean absolute error and of the mean relative error can be determined.

After this process is finished for all trainining techniques and all models suggested for each of the trainining techniques , the mean errors and standard deviations calculated by for the models suggested with any given training technique can be used as a key metric to judge the quality of the given training technique in view of the models suggested by the training model. In the embodiment shown, the key metric chosen to compare the techniques was the Mean Absolute Percentage Error (MAPE). The best performing model was found to be the MLP directly applied to the time series with MAPE=9%m with the second best model though is the Linear Regression model trained on the selected features that achieves 10% of MAPE.

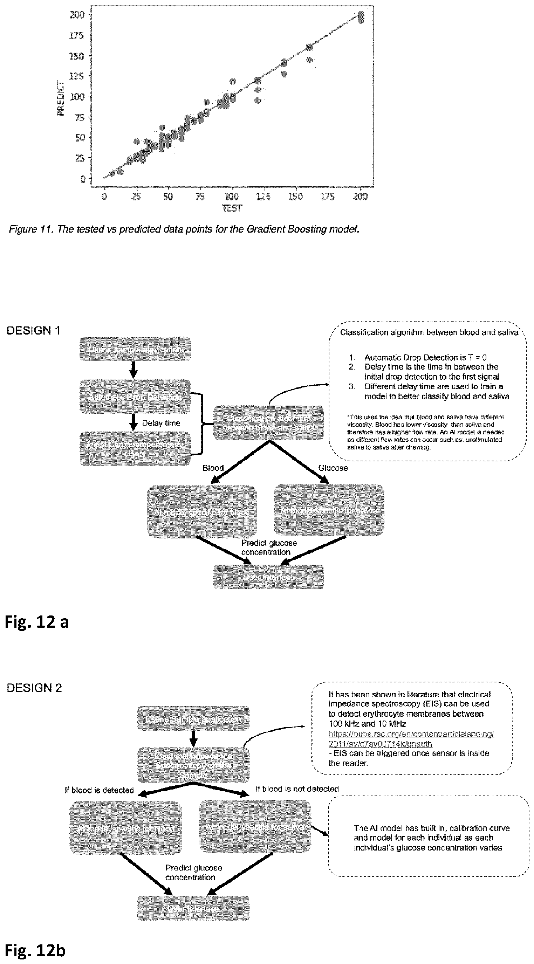

The relationship between predicted glucose concentration and actual concentration for the test set obtainable in this manner is shown in Fig. 11.

It will be understood that when considering two or more different body fluids such as blood and saliva, a best model can be established for each body fluid and once the body fluid is known, it is possible to select the respective correct model.

To this end, the present invention suggests to either establish a model for determination of the type of body fluid from a data set comprising samples obtained with both or all kinds of body fluid; once such determination has been made, the correct model for determining the analyte concentration can be used.

In another approach, the differentiation between different body fluids can be made using additional measurements such as electrical impedance spectroscopy as suggested above, even though this might require additianl time for data acquisition. The two different approaches disclosed by the present invention are shown in Fig. 12 a and Fig. 12b. Note that combinations or variations of these differentiation techniques are possible. For example, where the time body fluid is applied is determined, a signal delay time until a step response is observed in the signal obtained that can serve as an indicator weather sample is saliva or blood.

Variations of the methods and devices described are possible.

For example, in the embodiment above, it has been suggested that a number of linear coefficients are determined during calibration, but that the parameters /features) derived from the time series of signal values obtained are maintained the same regardless of carrier batch variations; it has been suggested to include the different linear coefficients in batch-specific information such as a batch-specific QR code that can be read out by the read-out device when using of a carrier from that batch. However, it will be understood that it would also be possible to determine whether a given set of parameters derived from the time series of signal values is an optimum set for a given batch. For example, for most carrier batches used in determining the glucose content of a body fluid, the following features derived from the time series of chronoamperometric values will be discarded: maximum value (peak value) of time series, mean value from the peak for 10 seconds, variations of autocorrelation coefficients, temporal characteristics of the peaks, width of a peak or the moment of its occurrence in the signal after starting a reaction. Nonetheless, in some batches at least one of such otherwise discarded features might prove to be more advantageous.

Thus, for the sake of completeness of the disclosure, it should be mentioned that it would also be possible to determine during calibration for a given batch, that some or all of these features or features normally not used would be a particularly good choice. It will be understood that this might increase the amount of information necessary for proper signal strength assessment, might increase the time necessary for the read-out device to adapt to the new calibration and might require a larger number of calibration measurements, in particular for carriers to be used with a multitude of body fluids where doubts exist whether a given a given set of parameters is equally suitable for all body fluids the carrier is intended for. Thus, the average skilled person will appreciate that very favorable results can be achieved by just batch-wise adaption of coefficients to a batch-independently fixed set of parameters (features) used in an assessment stage. This holds in particular where techniques such as neural filtering are used and the calibration coefficients are used as filter coefficients in the neural filtering.

It should be noted that while the claims when filing the application are directed to a read-out device comprising an interface adapted to obtain signals indicative of an analyte level of an analyte in a body fluid from a carrier adapted to receive in use the body fluid and to react in a detectable manner with the analyte and/or one or more concomitant substances in the body fluid; and a signal processing stage adapted to process the signals in a manner allowing determination of the level of the analyte in the body fluid is suggested, wherein the interface is adapted to obtain at least part of the signal as a time series of signal strengths from the carrier after receipt in use of one type of body fluid selectable from at least two different types with each type containing the analyte in a determinable amount and the signal processing stage is adapted to assess the analyte level from the time series of signal strengths for either of the types of body fluid received on the carrier, and hence relate to a multi-body-fluid analyzing device, aspects other than the fact that different body fluids can be used with one and the same strip may be considered inventive and may in particular be considered inventive per se.

In particular, it has been indicated that signals are obtained from a carrier having electrodes in an observation area, in particular electrodes that have been coated with enzyme(s) and mediator(s) so that a reaction takes place when the electrodes come into contact with a body fluid. It has been disclosed that a series of signals may be obtained in a chronoamperometric and/or electrical impedance measurement and that a reaction between components of a body fluid and reagents is in particular probed in the read-out device by applying specific voltage sequences, in particular of rectangular waveform having an amplitude that remains the same over the sequence or is, as is preferred, varied. The sequences are then analyzed as time series, deriving a plurality of features from the entire signal curve, the curve starting with and including the peak or at least the signal tail following some time after the peak to determine a concentration. This is considered inventive per se, even if e.g. the read-out device is adapted to only measure one analyte in one type of body fluid with a given type of carrier and if only one sort of carriers can be used. Applicant reserves the right to file divisional applications directed to such methods and/or devices. This holds in particular where from the time series, a number of features are used relating to at least one, preferably several and in particular all of the form of the signal curve, an area under the curve, a value of the autocorelation function at a specific time such as t=8, 9 or 10s, the decay (reduction) of the signal a specified first period after the peak, such as t= 0,5 or t= 1s or the decay (reduction) of the signal after some specified second period, such as e.g. 5s.

While applicant considers that obtaining and evaluating an entire, feature rich time series may be advantageous for a large number of analytes and body fluids and even independent on the specific waveform applied in chronometry or electrical impedance spectroscopy, a particularly preferred use is for the determination of the glucose level in saliva. A method for which applicant reserves the right to file divisional applications is believed to be advantageous over the prior inter alia because measurement and evaluation are fast and particularly good results are obtained for low concentration. It is believed that using the method of the present invention, despite the fact that signal acquisition currently lasts as long as 20 seconds or more, a time until a result is produced can be as short as 10 seconds or even 5 seconds. Also, as signals are measured directly and interferences are taken into account, the method is believed to yield more reliable results than previously known methods such as those relying on organic thin film transistors.

## Claims

1. A read-out device comprising

    an interface adapted to obtain
    signals indicative of an analyte level of an analyte in a body fluid from a carrier adapted to receive in use the body fluid and to react in a detectable manner with the analyte and/or one or more concomitant substances in the body fluid; and
    a signal processing stage
    adapted to process the signals in a manner allowing determination of the level of the analyte in the body fluid;

    wherein

    the interface is adapted to obtain at least part of

        the signal as a time series of signal strengths from the carrier after receipt in use of one type of body fluid selectable from at least two different types with each type containing the analyte in a determinable amount,
        and
        the signal processing stage is adapted to assess the analyte level from the time series of signal strengths for either of the types of body fluid received on the carrier.

2. The read-out device according to claim 1 wherein the interface is adapted obtain the signals from the carrier optically and/or electrochemically, preferably by chronoamperometry and/or by electrical impedance spectroscopy.

3. The read-out device according to the previous claim wherein the group of at least two different types of body fluid comprises at least one of (blood, urine, saliva) and preferably at least two of (blood, urine, saliva), in particular blood and saliva.

4. The read-out device according to any of the previous claims wherein the signal processing stage is adapted to store coefficients and/or parameters for at least two different models for assessing the level of analyte in response to the parameters, with each model relating to a different type of body fluid, the signal processing stage being adapted to select the appropriate model in view of the time series of values and/or in view of additional signals being obtained.

5. The read-out device according to the previous claims wherein the signal obtained is digitized and the signal processing stage is adapted to process a time series of digital values.

6. The read-out device according to the previous claim wherein the signal processing stage is adapted to evaluate the time series locally such that an indication of the level of analyte can be output.

7. The read-out device according to any of the previous claims wherein the signal processing stage is adapted to process a time series comprising at least 5 samples, preferably in particular at least 10 samples, particularly preferably at least 20 samples, taken over a period of time sufficient for observing the onset of a signal increase, in particular over a period of at least 2, preferably at least 5, in particular at least 10 seconds, and comprising samples obtaining following a maximum of a signal strength value and/or having a resolution after digitalization of at least 8, preferably at least 10 in particular at least 12 bit.

8. The read-out device according to any of the previous claims wherein the signal processing stage is adapted to assess the level of analyte in a quantitative manner.

9. The read-out device wherein

    the interface is further adapted to read out calibration information from the carrier and
    the signal processing stage is adapted to determine a plurality of parameters from the time series of digital signal values and to assess the level of analyte in response to both the parameters determined and the calibration information.

**10.** The read-out device according to any of the previous claim wherein the analyte is glucose or a concomitant indicator of glucose concentration.

**11.** The read-out device according to any of the previous claims wherein the signal processing stage is adapted to store parameters and/or coefficients for at least two different models for assessing the level of analyte in response to the parameters, with each model relating to a different type of body fluid, the signal processing stage being adapted to select the appropriate model in view of the time series of values in view of additional values being obtained.

**12.** The read-out device according to any of the previous claims adapted to obtain signals from disposable carriers.

**12.** The read-out device according to any of the previous claims having an interface to a smart device, in particular a smart phone, smart watch or a laptop.

**13.** A disposable carrier adapted for use with a read-out device according to any of the previous claims wherein the carrier is adapted to react with an analyte or a concomitant substance in the at least two types of body fluid and the interface is adapted to obtain the signal optically and/or electrochemically, preferably by chronoamperometry or by electrical impedance spectroscopy.

**14.** A disposable carrier according to claim 13, having electrodes for chronoamperometry, the electrodes being coated with reagents for glucose detection, the disposable carrier preferably comprising additional electrodes for the determination of a body fluid type by electrical impedance spectroscopy and the carrier having associated therewith or carrying calibration information relating to at least types of body fluid with one body fluid being blood.

Fig. 1

Fig. 2b

Fig. 2a

$$i = kt^{-1/2}$$

$$i = \left( \frac{nFAc_i^0 \sqrt{D_i}}{\sqrt{\pi t}} \right)$$

Fig. 3

Fig. 4a

Fig 4b

Fig. 5

Figure 6. Collection of the normalized data curves.

Fig. 7

Figure 8. The maximum value of the current, an example of a bad feature that has been rejected.

Figure 9. The overview of the training process for the second approach.

Figure 7. The features selected for ML training.

Fig. 10 a

Fig 10b

Figure 11. The tested vs predicted data points for the Gradient Boosting model.

DESIGN 1

Fig. 12 a

DESIGN 2

Fig. 12b

Europäisches
Patentamt

European
Patent Office

Office européen
des brevets

# EUROPEAN SEARCH REPORT

Application Number

EP 23 02 0052

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/200697 A1 (EL MURR NABIL [FR] ET AL) 25 June 2020 (2020-06-25)<br>* the whole document *<br>----- | 1-14 | INV.<br>A61B5/145 |
| X | US 2015/253321 A1 (CHOU STEPHEN Y [US] ET AL) 10 September 2015 (2015-09-10)<br>* paragraph [0004] *<br>* paragraph [0036] *<br>* paragraph [0068] – paragraph [0083] *<br>* paragraph [0161] – paragraph [0164] *<br>* paragraph [0191] – paragraph [0198] *<br>* figures 1-12 *<br>----- | 1-14 | |
| X | US 2020/367808 A1 (NATH RATTAN [US] ET AL) 26 November 2020 (2020-11-26)<br>* paragraph [0011] – paragraph [0019] *<br>* paragraph [0106] – paragraph [0143] *<br>* figures 1-10 *<br>----- | 1-14 | |

TECHNICAL FIELDS
SEARCHED (IPC)

A61B

The present search report has been drawn up for all claims

| Place of search | Date of completion of the search | Examiner |
|---|---|---|
| Berlin | 3 July 2023 | Abraham, Volkhard |

EPO FORM 1503 03.82 (P04C01)

## ANNEX TO THE EUROPEAN SEARCH REPORT
## ON EUROPEAN PATENT APPLICATION NO.

EP 23 02 0052

This annex lists the patent family members relating to the patent documents cited in the above-mentioned European search report.
The members are as contained in the European Patent Office EDP file on
The European Patent Office is in no way liable for these particulars which are merely given for the purpose of information.

03-07-2023

| Patent document cited in search report | | Publication date | Patent family member(s) | | Publication date |
|---|---|---|---|---|---|
| US 2020200697 | A1 | 25-06-2020 | BR | 112019025079 A2 | 16-06-2020 |
| | | | CN | 111065762 A | 24-04-2020 |
| | | | EP | 3635155 A1 | 15-04-2020 |
| | | | JP | 2020523565 A | 06-08-2020 |
| | | | US | 2020200697 A1 | 25-06-2020 |
| | | | WO | 2018226686 A1 | 13-12-2018 |
| US 2015253321 | A1 | 10-09-2015 | CN | 104823049 A | 05-08-2015 |
| | | | EP | 2904389 A1 | 12-08-2015 |
| | | | US | 2015253321 A1 | 10-09-2015 |
| | | | WO | 2014055559 A1 | 10-04-2014 |
| US 2020367808 | A1 | 26-11-2020 | US | 2017258395 A1 | 14-09-2017 |
| | | | US | 2020367808 A1 | 26-11-2020 |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1986007 A1
- EP 0199484 A2
- EP 0840122 A
- EP 1972270 A1
- EP 2924436 A1
- KR 101436915
- US 20160313307 A1
- WO 2014055559 A1
- CA 2732786 A1
- US 20070106138 A1
- US 20080020477 A1
- US 20160097734 A1
- US 20190025131 A1
- WO 2017058806 A1
- WO 2013131130 A1
- WO 201914415581 A
- US 9766199 B2
- WO 2018000012 A
- WO 2019218011 A
- EP 3220138 A1
- JP 2002122562 A
- US 20070233395 A1
- WO 2005080970 A
- WO 2017116503 A

**Non-patent literature cited in the description**

- **MING XING CHU ; KUMIKO MIYAJIMA ; DAISHI TAKAHASHI ; TAKAHIRO ARAKAWAB ; KENJI SANO ; SHIN-ICHI SAWADA ; HIROYUKI KUDOB ; YASUHIKO IWASAKID ; KAZUNARI AKIYOSHI ; MANABU MOCHIZUKI.** Soft contact lens biosensor for in situ monitoring of tear glucose as non-invasive blood sugar assessment. *Talanta,* 2011, vol. 83, 960-965
- **TAKAHIRO ARAKAWA ; YUSUKE KUROKI ; HIROKI NITTA ; PREM CHOUHAN ; KOJI TOMA ; SHIN-ICHI SAWADA ; SHUHEI TAKEUCHI ; TOSHIAKI SEKITA ; KAZUNARI AKIYOSHI ; SHUNSUKE MINAKUCHI.** *Mouthguard biosensor with telemetry system for monitoring of saliva glucose: A novel cavitas sensor, http://dx.doi.org/10.1016/j.bios.2015.12.014*
- **KOJI TOMA ; DAISUKE MIKI ; NAOYUKI YOSHIMURA ; TAKAHIRO ARAKAWA ; HIROMI YATSUDA ; KOHJI MITSUBAYASHI.** *A gold nanoparticle-assisted sensitive SAW (surface acoustic wave) immunosensor with a regeneratable surface for monitoring of dust mite allergens, http://dx.doi.org/10.1016/j.snb.2017.04.073*
- **PO-JEN CHIEN ; TAKUMA SUZUKI ; MASATO TSUJII ; MING YE ; ISAO MINAMI ; KANAKO TODA ; HIROMI OTSUKA ; KOJI TOMA ; TAKAHIRO ARAKAWA ; KOUJI ARAKI.** Biochemical Gas Sensors (Biosniffers) Using Forward and Reverse Reactions of Secondary Alcohol Dehydrogenase for Breath Isopropanol and Acetone as Potential Volatile Biomarkers of Diabetes Mellitus. *Anal. Chem.,* 2017, vol. 89, 12261-12268
- **XENOFON STRAKOSAS ; JOHN SELBERG ; PATTAWONG PANSODTEE ; NEBYU YONAS ; PATTAWUT MANAPONGPUN ; MIRCEA TEODORESCU ; MARCO ROLANDI.** A non-enzymatic glucose sensor enabled by bioelectronic pH control. *Scientific Reports,* 2019, vol. 9, 10844, https://doi.org/10.1038/s41598-019-46302-9
- **SARUL MALIK ; HARSH PARIKH ; NEIL SHAH ; SNEH ANAND ; SHALINI GUPTA.** Non-invasive platform to estimate fasting blood glucose levels from salivary electrochemical parameters. *Healthcare Technology Letters,* 2019, vol. 6 (4), 87-91
- **KAROLINA ELZBIETA KACZOR-URBANOWICZ ; CARMEN MARTIN CARRERAS-PRESAS ; KATRI ARO ; MICHAEL TU ; FRANKLIN GARCIA-GODOY ; DAVID TW WONG.** Saliva diagnostics - Current views and directions. *Experimental Biology and Medicine,* 2017, vol. 242, 459-472
- **PAULO MASCARENHAS ; BRUNO FATELA ; ISABEL BARAHONA.** Effect of Diabetes Mellitus Type 2 on Salivary Glucose - A Systematic Review and Meta-Analysis of Observational Studies. *PLOS ONE,* 01 July 2014, vol. 9 (7), e101706
- **KOMAL SMRITI ; KEERTHILATHA M. PAI ; VINEETHA RAVINDRANATH ; SRIKANTH GADICHERLA ; KALYANA CHAKRAVARTHY PENTAPATI.** *Salivary Glucose as a Diagnostic Marke for Diabetes Mellitus*

- **JAYOUNG KIM ; SOMAYEH IMANI ; WILLIAM R. DE ARAUJO ; JULIAN WARCHALL ; GABRIELA VALDÉS-RAMÍREZ ; THIAGO R.L.C. PAIXÃO ; PATRICK P. MERCIER ; JOSEPH WANG.** Wearable salivary uric acid mouthguard biosensor with integrated wireless electronics. *Biosensors and Bioelectronics,* 2015, vol. 74, 1061-1068
- **WENJUN ZHANG ; YUNQING DU ; MING L. WANG.** Noninvasive glucose monitoring using saliva nano-biosensor. *Sensing and Bio-Sensing Research,* 2015, vol. 4, 23-29
- **WENJUN ZHANG ; YUNQING DU ; MING L. WANG.** On-chip highly sensitive saliva glucose sensing using multilayer films composed of single-walled carbon nanotubes, gold nanoparticles, and glucose oxidase. *Sensing and Bio-Sensing Research,* 2015, vol. 4, 96-102
- **SHREYA GUPTA ; MEGHANAND T NAYAK ; JD SUNITHA ; GEETANSHU DAWAR ; NIDHI SINHA ; NEELAKSHI SINGH RALLAN.** Correlation of salivary glucose level with blood glucose level in diabetes mellitus. *JOMFP,* 2017, vol. 21 (3), 334-339
- **LAURA GARCIA-CARMONA ; AIDA MARTIN ; JULIANE R. SEMPIONATTO ; JOSE R. MORETO ; MARIA CRISTINA GONZÁLEZ ; JOSEPH WANG ; ALBERTO ESCARPA.** Pacifier Biosensor: Toward Noninvasive Saliva Biomarker Monitoring. *Anal. Chem.,* 2019, vol. 91, 13883-13891
- **YUNQING DU ; WENJUN ZHANG ; MING L. WANG.** *Sensing of Salivary Glucose Using Nano-Structured Biosensors*
- **GUY H. CARPENTER.** The Secretion, Components and Properties of Saliva. *Annu. Rev. Food Sci. Technol.,* 2013, vol. 4, 267-76
- **STEPAN PODZIMEK ; LUCIE VONDRACKOVA ; JANA DUSKOVA ; TATJANA JANATOVA ; ZDENEK BROUKAL.** Salivary Markers for Periodontal and General Diseases. *Disease Markers,* vol. 2016, http://dx.doi.org/10.1155/2016/9179632
- **DAE-WOONG HWANG ; SARAM LEE ; MINJEE SEO ; TAEK DONG CHUNG.** Recent advances in electrochemical non-enzymatic glucose sensors -a review. *Analytica Chimica Acta,* 2018, vol. 1033, 1-34
- **PANDA ABIKSHYEET ; VENKATAPATHY RAMESH ; NIRIMA OZA.** Glucose estimation and the salivary secretion of diabetes mellitus patients. *Diabetes, Metabolic Syndrome and Obesity: Targets and Therapy,* 2012, vol. 5, 149-154
- **PING-PING HAN ; SAŠO IVANOVSKI.** Effect of Saliva Collection Methods on the Detection of Periodontium-Related Genetic and Epigenetic Biomarkers-A Pilot Study. *Int. J. Mol. Sci.,* 2019, vol. 20, 4729
- **PHILIP D. MARSH ; THUY DO ; DAVID BEIGHTON ; DEIRDRE A. DEVINE.** influence of saliva on the oral microbiota. *Periodontology,* 2000, 80-92
- **MANU S. MANNOOR1 ; HU TAO2 ; JEFFERSON D. CLAYTON ; AMARTYA SENGUPTA ; DAVID L. KAPLAN ; RAJESH R. NAIK ; NAVEEN VERMA ; FIORENZO G. OMENETTO ; MICHAEL C. MCALPINE.** Graphene-based wireless bacteria detection on tooth enamel. *nature communications,* vol. 3 (7), 6-3
- **CHE-WEI HSU ; FANG-CI SU ; PO-YU PENG ; HONG-TSU YOUNG ; STEPHEN LIAO ; GOU-JEN WANG.** Highly sensitive non-enzymatic electrochemical glucose biosensor using a photolithography fabricated micro/nano hybrid structured electrode. *Sensors and Actuators B,* 2016, vol. 230, 559-565
- **RICHARD K. D. EPHRAIM ; ENOCH ODAME ANTO ; EMMANUEL ACHEAMPONG ; LINDA AHENKORAH FONDJO ; RICHMOND B. BARNIE ; SAMUEL ASAMOAH SAKYI ; AMBROSE ASARE.** Fasting salivary glucose levels is not a better measure for identifying diabetes mellitus than capillary blood glucose levels: comparison in the Ghanian population. *Heliyon,* 2019, vol. 5, e01286